(19) Europäisches Patentamt

European Patent Office

Office européen des brevets

(11) **EP 4 596 039 A1**

(12) **EUROPEAN PATENT APPLICATION**

(43) Date of publication:
**06.08.2025 Bulletin 2025/32**

(21) Application number: **25154462.3**

(22) Date of filing: **28.01.2025**

(51) International Patent Classification (IPC):
**A61N 5/10** $^{(2006.01)}$

(52) Cooperative Patent Classification (CPC):
**A61N 5/103;** A61N 2005/1041

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.01.2024 US 202418429136**

(71) Applicant: **Siemens Healthineers International AG 6312 Steinhausen (CH)**

(72) Inventors:
- **GAO, Riqiang**
  **Plainsboro, 08536 (US)**
- **GHESU, Florin-Cristian**
  **91083 Baiersdorf (DE)**
- **LOU, Bin**
  **Princeton Junction, 08550 (US)**
- **KAMEN, Ali**
  **Skillman, 08558 (US)**
- **COMANICIU, Dorin**
  **Princeton, 08540 (US)**
- **ARBERET, Simon**
  **Princeton, 08540 (US)**

(74) Representative: **Mathisen & Macara LLP Charta House 30-38 Church Street Staines-upon-Thames TW18 4EP (GB)**

(54) **SYSTEMS AND METHODS FOR PLANNING RADIATION THERAPY**

(57) Provided herein are methods and systems for planning radiation therapy. In examples, at least one processor can be programmed to: receive 202 data associated with a first planning target volume and a first radiation map, provide 204 the first planning target volume and the first radiation map to a first model to cause the first model to output data associated with at least one first beam position and least one first beam strength, generate 206 a second radiation map, and provide 208 the first planning target volume and the second radiation map to a second model to cause the second model to output data associated with at least one second beam position and least one second beam strength. At least one processor can be further programmed to: transmit 210 data associated with the at least one second beam position and the least one second beam strength to cause a linear accelerator to deliver radiation.

FIG. 2A

EP 4 596 039 A1

**Description**

## TECHNICAL FIELD

This application relates generally to systems and methods involved in planning radiation therapy using medical devices that deliver energy to patients.

## BACKGROUND

Radiotherapy is one of the most common treatments for cancers in the body. However, one of the major challenges in radiation therapy is planning the delivery of energy to volumes of tissue in the body such that the energy delivery is optimized and healthy tissue damage is minimized. For example, when using a linear accelerator to deliver energy, clinicians need to carefully configure treatment plans that maximize the delivery of energy to a tumor while at the same time minimizing delivery of energy to tissue around the tumor, which is otherwise healthy tissue.

Methods and systems available today for optimizing radiation therapy planning focus on the data available from patients on a case-by-case basis. For example, CT scans may be initially generated for a patient, and clinicians may provide input to guide a system controlling a linear accelerator when delivering energy to a planning target volume of the patient. The input may specify where beams are to be positioned, the intensity of the beams, and so on. This is an extremely time-consuming process and relies on each clinician developing expertise and improving over time. And conventional approaches to automating aspects of the planning process generally rely on brute-force methods when analyzing the CT scans of the patient and determining where beams are to be positioned as well as the intensity of the beams. This is both time-consuming and highly computational-resource intensive.

## SUMMARY

For the aforementioned reasons, there is a desire for a system that can improve radiation therapy planning. For instance, there is a desire to improve the radiation therapy planning process such that radiation therapy plans developed to take advantage of improving linear accelerator technology are more precise, optimizing the delivery of energy to planning target volumes while minimizing energy delivery to surrounding tissue. There is also a desire for techniques that enable consistent development of radiation therapy plans across varying patients having varying planning target volumes.

Aspects of the present disclosure relate to systems, methods, devices, apparatus, and non-transitory machine-readable media for planning radiation therapy. In embodiments, at least one processor is programmed to: receive data associated with a first planning target volume and a first radiation map, the first planning target volume representing an area of a body of a patient and a target mask, the target mask representing a portion of the area of the body of the patient to receive radiation; provide the first planning target volume and the first radiation map to a first model to cause the first model to output data associated with at least one first beam position and least one first beam strength, the at least one first beam position corresponding to the at least one first beam strength, generate a second radiation map based on the at least one first beam position, the at least one first beam strength, and the first radiation map; provide the first planning target volume and the second radiation map to a second model to cause the second model to output data associated with at least one second beam position and least one second beam strength. The data associated with the at least one second beam position and the least one second beam strength may be transmitted to a medical device and may be usable by the medical device to cause the medical device to deliver radiation to the patient. The medical device may be a linear accelerator (LINAC).

The first model may be trained to output data associated with first beam positions at a first scale, and the second model may be trained to output data associated with second beam positions at a second scale. The first scale may be associated with a first set of candidate angles within a candidate angle space, the second scale may be associated with a second set of candidate angles within the candidate angle space, and the first set of candidate angles may be associated with angle measurements that are greater than angle measurements associated with the second set of candidate angles. A beam associated with the second set of candidate angles corresponds to a beam associated with the first set of candidate angles.

The first model may be trained based on a reinforcement learning agent and training data associated with previously-treated patients. The training data may represent planning target volumes and goal radiation maps that correspond to the previously-treated patients. When training, the at least one processor may be further programmed to: for each previous-treated patient of the previously-treated patients: generate data associated with beam positions and beam strengths based on a plurality of reinforcement learning agents, each reinforcement learning agent associated with a training model, provide the data associated with beam positions and beam strengths to a simulator to generate a set of simulated outputs, determine rewards for each reinforcement learning agent of the plurality of reinforcement learning agents based on the corresponding simulated outputs and the goal radiation map, and select the first model based on the rewards for each reinforcement learning agent. The goal radiation maps represent radiation maps that were previously generated based on input by a clinician, the goal radiation maps representing preferences of the clinician when delivering radiation to the previously-treated patients.

In another embodiment, a method comprises: receiving, by at least one processor, data associated with a first planning target volume and a first radiation map, the first planning target volume representing an area of a body of a patient and a target mask, the target mask representing a portion of the area of the body of the patient to receive radiation; providing, by the at least one processor, the first planning target volume and the first radiation map to a first model to cause the first model to output data associated with at least one first beam position and least one first beam strength, the at least one first beam position corresponding to the at least one first beam strength, generating, by the at least one processor, a second radiation map based on the at least one first beam position, the at least one first beam strength, and the first radiation map; providing, by the at least one processor, the first planning target volume and the second radiation map to a second model to cause the second model to output data associated with at least one second beam position and least one second beam strength. The method may be a method of radiation treatment planning. The data associated with the at least one second beam position and the least one second beam strength may be transmitted to a medical device and may be usable by the medical device to cause the medical device to deliver radiation to the patient. The medical device may be a linear accelerator (LINAC).

In yet another embodiment, a non-transitory machine-readable medium having instructions stored thereon that, when executed by at least one processor, cause the at least one processor to perform operations comprising: receiving data associated with a first planning target volume and a first radiation map, the first planning target volume representing an area of a body of a patient and a target mask, the target mask representing a portion of the area of the body of the patient to receive radiation; providing the first planning target volume and the first radiation map to a first model to cause the first model to output data associated with at least one first beam position and least one first beam strength, the at least one first beam position corresponding to the at least one first beam strength, generating a second radiation map based on the at least one first beam position, the at least one first beam strength, and the first radiation map; providing the first planning target volume and the second radiation map to a second model to cause the second model to output data associated with at least one second beam position and least one second beam strength. The data associated with the at least one second beam position and the least one second beam strength may be transmitted to a medical device and may be usable by the medical device to cause the medical device to deliver radiation to the patient. The medical device may be a linear accelerator (LINAC).

By virtue of implementing the above-described techniques, systems and methods may incorporate machine learning-based techniques to processes for planning and delivering radiation therapy. In practice, patients are prescribed a specific radiation dose for their tumors and this dose delivery is distributed over multiple days (or fractions). A common variant in radiotherapy is the image-guided radiotherapy (IGRT), where daily (Cone Beam, Mega Voltage, or a low-Tesla MR) images of the patient anatomy are acquired for verification of the patient setup before each treatment fraction. Additionally, intensity modulated RT (IMRT) and Volumetric Arc Therapy (VMAT) are techniques that ensure that the radiation dose delivery is tailored closely to the target. By implementing the above-noted techniques, the dose delivery of radiation to the planning target volume can be optimized, the dose delivery of radiation to organs or structures at risk in proximity to the planning target volume can be consistently minimized, and the planned therapy can be confirmed as being deliverable (in terms of the machine capabilities) and efficiently (in a shortest amount of time) as compared to other techniques such as modeling (e.g., modeling prior to optimization (e.g., utilizing a cost function that can map the relative importance of different criteria), modeling during the optimization (e.g., allowing clinicians to actively interact with the optimization and adjust the relative importance of one criterion versus another (e.g., treatment duration versus radiation to organs at risk)), and modeling after optimization (e.g., creating a pareto-surface a priori by re-running the optimization with different configurations and multi-criteria dynamics.

Aspects of the present disclosure relate to systems, methods, devices, apparatus, and non-transitory machine-readable media for generating fluence maps when planning radiation therapy. In embodiments, at least one processor programmed to: receive data associated with a plurality of beams, each beam of the plurality of beams specifying a beam angle measured relative to a LINAC; generating beam eye view (BEV) images based on the plurality of beams; determining a digitally reconstructed radiograph (DRR) based on concatenating the BEV images; and providing the DRR to a model to cause the model to generate an output, the output associated with a fluence map.

When providing the DRR to the model, the at least one processor may be programmed to: provide the DRR to at least one encoder along a contracting path of the model to cause the at least one encoder to provide a first output to at least one second encoder and a second output to at least one decoder corresponding to the at least one encoder, wherein the at least one decoder is along an expansive path of the model. When providing the first output to the at least one second encoder, the at least one processor is programmed to: provide the first output to the at least one second encoder to cause the at least one second encoder to: provide a third output to at least one second decoder along the expansive path of the model, the at least one second decoder corresponding to the at least one second encoder, wherein the second decoder is configured to provide a fourth output to the at least one decoder based on the third output.

When receiving the data associated with a plurality of beams, the at least one processor is programmed to: receive data associated with positions of a plurality of leaves corresponding to each beam of the plurality of beams, the plurality of positions representing a configuration of the plurality leaves, and wherein, when generating the BEV images, the at least one processor is programmed to: generate the BEV images based on delivery of radiation by the linac while the plurality of leaves are in the configuration. When generating the BEV images, the at least one processor may be programmed to: generate the BEV images based on power levels at which the linac delivers radiation.

When receiving the data associated with the plurality of leaves, the at least one processor may be programmed to: receive data associated with positions of the plurality of leaves across a plurality of time steps for each beam of the plurality of beams, where the positions of the plurality of leaves at each time step is associated with a sub-configuration of the plurality of leaves, and when generating the BEV images, the at least one processor may be programmed to: generate each BEV image based on delivery of radiation by the linac at each time step of the plurality of time steps. When generating the BEV images, the at least one processor may be programmed to: generate the BEV images based on power levels at which the linac delivers radiation for each beam of the plurality of beams.

In yet another embodiment, a non-transitory machine-readable medium having instructions stored thereon that, when executed by at least one processor, cause the at least one processor to perform operations comprising: receiving data associated with a plurality of beams, each beam of the plurality of beams specifying a beam angle measured relative to a LINAC; generating BEV images based on the plurality of beams; determining a DRR based on concatenating the BEV images; and providing the DRR to a model to cause the model to generate an output, the output associated with a fluence map.

In another embodiment, a method comprises: receiving, by at least one processor, data associated with a plurality of beams, each beam of the plurality of beams specifying a beam angle measured relative to a LINAC; generating, by the at least one processor, BEV images based on the plurality of beams; determining, by the at least one processor, a DRR based on concatenating the BEV images; and providing, by the at least one processor, the DRR to a model to cause the model to generate an output, the output associated with a fluence map.

By virtue of implementing the above-described techniques, systems and methods may adopt machine learning-based approaches to the development of fluence maps which improve on technologies such as inverse planning for fluence map development. The disclosed techniques can result in faster fluence map development which, in turn, can shorten the overall radiation therapy planning process. As a result, the power and computing resources can be conserved and/or redirected to the treatment of additional patients. Further, the presently-disclosed techniques allow for the careful processing of multiple beams without access to field-dose distributions per dose to support the training process.

Aspects of the present disclosure relate to systems, methods, devices, apparatus, and non-transitory machine-readable media for generating leaf sequences when planning radiation therapy. In embodiments, at least one processor programmed to: receive input data associated with a target fluence map, the target fluence map representing a goal amount of radiation to deliver to a patient via a LINAC; provide the input data to a model to cause the model to generate an output, the output representing a cumulative fluence map and a leaf mask; and while a difference between the cumulative fluence map and the target fluence map satisfies a first threshold amount: generate updated input data associated with the target fluence map, the cumulative fluence map, and the leaf mask, provide the updated input data to the model to cause the model to generate an updated output, the updated output representing an updated cumulative fluence map and an updated leaf mask, determine that the difference between the updated cumulative fluence map and the target fluence map satisfies a second threshold amount; and determine a leaf sequence based on the leaf mask and the updated leaf mask.

When determining the leaf sequence, the at least one processor may be programmed to: determine a difference between the leaf mask and the updated leaf mask; and determine the leaf sequence based on the difference between the leaf mask and the updated leaf mask.

When determining the difference between the leaf mask and the updated leaf mask, the at least one processor may be programmed to: determine a first leaf position and a second leaf position associated with a leaf pair of the linac, the first leaf position and the second leaf position corresponding to the leaf mask; and determine a third leaf position and a fourth leaf position associated with the leaf pair, the third leaf position and the fourth leaf position corresponding to the updated leaf mask, wherein the difference between the leaf mask and the updated leaf mask is represented by a change in position between the first leaf position and the third leaf position, and a change in position between the second leaf position and the fourth leaf position.

When determining the difference between the leaf mask and the updated leaf mask, the at least one processor may be programmed to: determine a first rate of motion for a first leaf of a leaf pair during a time period; determine a second rate of motion for a second leaf of the leaf pair during the time period; and determine the difference between the leaf mask and the updated leaf mask based on the first rate of motion for the first leaf and the second rate of motion for the second leaf.

When determining the difference between the leaf mask and the updated leaf mask, the at least one processor may be programmed to: determine at least one radiation dose to deliver via the LINAC as the leaf pair transitions from the leaf mask to the updated leaf mask. The LINAC may comprise a plurality of leaf pairs, and the model may comprise a plurality of agent policies, each agent policy of the plurality of agent policies corresponding to a leaf pair of the plurality of leaf pairs.

Each agent policy may be trained based on training data associated with a plurality of previously-treated patients, the training data comprising a plurality of target fluences. The at least one processor may be further programmed to: for each agent of a plurality of agents corresponding to a leaf pair and each target fluence: generate, by each agent, motion data associated with motion of the leaf pair and radiation doses to deliver via the linac as the leaf pair moves over a plurality of time steps, provide, for each agent, the motion data to a simulator to generate simulated fluence maps for each time step of the plurality of time steps, determine rewards for each agent based on the simulated fluence maps, and select a final agent from among the plurality of agents based on scores for each agent of the plurality of agents, the scores corresponding to cumulative scores for each agent of the plurality of agents.

In yet another embodiment, a non-transitory machine-readable medium having instructions stored thereon that, when executed by at least one processor, cause the at least one processor to perform operations comprising: providing the input data to a model to cause the model to generate an output, the output representing a cumulative fluence map and a leaf mask; and while a difference between the cumulative fluence map and the target fluence map satisfies a first threshold amount: generating updated input data associated with the target fluence map, the cumulative fluence map, and the leaf mask, providing the updated input data to the model to cause the model to generate an updated output, the updated output representing an updated cumulative fluence map and an updated leaf mask, determining that the difference between the updated cumulative fluence map and the target fluence map satisfies a second threshold amount; and determining a leaf sequence based on the leaf mask and the updated leaf mask.

In another embodiment, a method comprises: receiving, by at least one processor, input data associated with a target fluence map, the target fluence map representing a goal amount of radiation to deliver to a patient via a LINAC; providing, by the at least one processor, the input data to a model to cause the model to generate an output, the output representing a cumulative fluence map and a leaf mask; and while a difference between the cumulative fluence map and the target fluence map satisfies a first threshold amount: generating, by the at least one processor, updated input data associated with the target fluence map, the cumulative fluence map, and the leaf mask, providing, by the at least one processor, the updated input data to the model to cause the model to generate an updated output, the updated output representing an updated cumulative fluence map and an updated leaf mask, determining, by the at least one processor, that the difference between the updated cumulative fluence map and the target fluence map satisfies a second threshold amount; and determining, by the at least one processor, a leaf sequence based on the leaf mask and the updated leaf mask.

By virtue of the implementation of the techniques described herein, leaf sequencing can be formulated as a decision-making problem suitable to be addressed by deep reinforcement learning (DRL) techniques described herein. For example, leaf sequencing models can be implemented to handle different plan types (VMAT and IMRT). These models can also improve the overall time needed to compute a leaf sequence, again reducing the need for computational resources and power which can either be conserved or directed to address additional patients.

## BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.

FIG. 1 illustrates a diagram of a system for planning and delivering radiation therapy, in accordance with an embodiment.

FIG. 2A illustrates a diagram of process for planning radiation therapy, in accordance with an embodiment.

FIGS. 2B-2D illustrate a flowchart of an implementation relating to a process for planning and delivering radiation therapy, in accordance with an embodiment.

FIG. 3A illustrates a diagram of a process for generating fluence maps when planning radiation therapy, in accordance with an embodiment.

FIGS. 3B-3D illustrate a flowchart of an implementation relating to a process for generating fluence maps when planning radiation therapy, in accordance with an embodiment.

FIG. 4A illustrates a diagram of a process for generating leaf sequences when planning radiation therapy, in accordance with an embodiment.

**FIGS. 4B-4F** illustrate a flowchart of an implementation relating to a process for generating leaf sequences when planning radiation therapy, in accordance with an embodiment.

**FIG. 4G** illustrates one example iteration of the implementation of **FIGS. 4B-4F.**

**FIG. 4H** illustrates example pairs of predicted fluence maps and target (e.g., cumulative) fluence maps, in accordance with an embodiment.

**FIGS. 5A and 5B** illustrate examples training pipelines involving multiple systems, in accordance with an embodiment.

## DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are to be considered within the scope of the subject matter disclosed herein. Other embodiments may be used, and/or other changes may be made without departing from the spirit or scope of the present disclosure. The \illustrative embodiments described in the detailed description are not meant to be limiting of the subject matter presented.

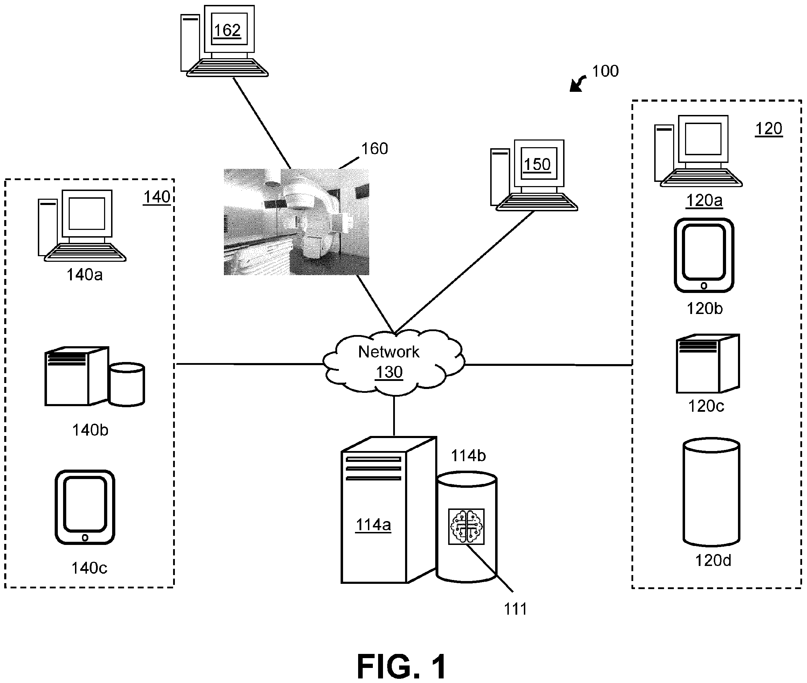

**FIG. 1** illustrates components of a system **100** for planning and delivering radiation therapy, according to an embodiment. The system **100** may include an analytics server **114a,** system database **114b,** an AI model **111,** electronic data sources **120a-d** (collectively electronic data sources **120),** end-user devices **140a-c** (collectively end-user devices **140**), an administrator computing device **150,** a medical device **160,** and medical device computer(s) **162.** Various components depicted in **FIG. 1** may belong to a radiotherapy clinic at which patients may receive radiotherapy treatment, in some cases via one or more radiotherapy machines located within the clinic (e.g., medical device **160**). The system **100** is not confined to the components described herein and may include additional or other components, not shown for brevity, which are to be considered within the scope of the embodiments described herein.

The above-mentioned components may be connected to each other through a network **130.** Examples of the network **130** may include, but are not limited to, private or public local-area-networks (LAN), wireless LAN (WLAN) networks, metropolitan area networks (MAN), wide-area networks (WAN), and the Internet. The network 130 may include wired and/or wireless communications according to one or more standards and/or via one or more transport mediums. The communication over the network **130** may be performed in accordance with various communication protocols such as Transmission Control Protocol and Internet Protocol (TCP/IP), User Datagram Protocol (UDP), and IEEE communication protocols. In one example, the network **130** may include wireless communications according to Bluetooth specification sets or another standard or proprietary wireless communication protocol. In another example, the network **130** may also include communications over a cellular network, including, e.g., a GSM (Global System for Mobile Communications), CDMA (Code Division Multiple Access), and EDGE (Enhanced Data for Global Evolution) network.

The analytics server **114a** may generate and display an electronic platform configured to use an AI model **111** (including artificial intelligence and/or machine learning models) for receiving patient information and outputting the results of execution of the AI model **111.** The electronic platform may include graphical user interfaces (GUI) displayed on one or more electronic data sources **120,** the end-user devices **140,** the medical device **160,** and/or the administrator computing device **150.** An example of the electronic platform generated and hosted by the analytics server **114a** may be a web-based application or a website configured to be displayed on different electronic devices, such as mobile devices, tablets, personal computers, and the like.

The information displayed by the electronic platform can include, for example, input elements to receive data associated with a patient being treated, synchronize one or more sensors, and display results of predictions produced by the AI model **111.** For instance, the analytics server **114a** may execute the AI model **111** (e.g., machine learning models trained to generate fluence maps, leaf sequences, etc., as described herein for a patient being treated via the medical device **160**). The analytics server **114a** may then display the results for a clinician and/or directly revise one or more operational attributes of the medical device **160.**

The analytics server **114a** may be any computing device comprising a processor and non-transitory machine-readable storage capable of executing the various tasks and processes described herein. The analytics server **114a** may employ various processors such as central processing units (CPU) and graphics processing unit (GPU), among others. Non-limiting examples of such computing devices may include workstation computers, laptop computers, server computers, and the like. While the system **100** includes a single analytics server **114a,** the analytics server **114a** may include any number of computing devices operating in a distributed computing environment, such as a cloud environment.

The electronic data sources **120** may represent various electronic data sources that contain, retrieve, and/or access data associated with a medical device **160,** such as operational information associated with previously performed radiotherapy treatments (e.g., electronic log files or electronic configuration files), data associated with previously monitored patients (e.g., CT scans, tumor locations, deformation information, and/or the like) or participants in a study to train the AI models discussed herein. For instance, the analytics server **114a** may use the clinic computer **120a,** medical professional device **120b,** server **120c** (associated with a clinician and/or a clinic), and database **120d** (associated with the clinician and/or the clinic) to retrieve/receive data associated with the medical device **160**. The analytics server **114a** may retrieve the data from the end-user devices **120,** generate a training dataset, and train the AI models **111**. The analytics server **114a** may execute various algorithms to translate raw data received/retrieved from the electronic data sources **120** into machine-readable objects that can be stored and processed by other analytical processes as described herein.

End-user devices **140** may be any computing device comprising a processor and a non-transitory machine-readable storage medium capable of performing the various tasks and processes described herein. Non-limiting examples of an end-user device **140** may be a workstation computer, laptop computer, tablet computer, or server computer. In operation, various users may use end-user devices **140** to access the GUI operationally managed by the analytics server **114a** or otherwise the results of the execution of the AI model **111**. Specifically, the end-user devices **140** may include clinic computer **140a,** clinic server **140b,** and a medical professional device **140c**. Even though referred to herein as "end-user" devices, these devices may not always be operated by end-users. For instance, the clinic server **140b** may not be directly used by an end user. However, the results stored on the clinic server **140b** may be used to populate various GUIs accessed by an end user via the medical professional device **140c**. In some embodiments, the end-user device **140** may be associated with one or more clinicians that are associated with one or more treatment plans (e.g., involved in preparing the one or more treatment plans) for patients).

The administrator computing device **150** may represent a computing device operated by a system administrator. The administrator computing device **150** may be configured to display radiotherapy treatment attributes generated by the analytics server **114a** (e.g., various analytic metrics determined during training of one or more machine learning models and/or systems); monitor various models **111** utilized by the analytics server **114a,** electronic data sources **120,** and/or end-user devices **140;** review feedback; and/or facilitate training or retraining (calibration) of the AI model **111** that are maintained by the analytics server **114a.**

In some embodiments, the medical device **160** can be a diagnostic imaging device or a treatment delivery device. For example, the medical device 160 may include one or more computed tomography (CT) scanners, linear accelerators (LINACs) having a multi-leaf collimator (referred to herein as a collimator for ease of description) that consists of multiple small lead leaves that can be individually moved to shape the radiation beam and deliver the dose to the tumor while minimizing the dose to surrounding healthy tissues, or other similar devices configured to transmit energy toward targeted tissue (referred to as planning target volumes) associated with a patient and, in some cases, measure the energy transferred to ward the targeted tissue. The medical device **160** may also include one or more sensors configured to monitor the patient being treated. That is, the medical device **160** and/or the analytics server **114a** may be communicating with various sensors that can monitor a patient's external biological signals. Non-limiting examples of the sensors may include 3D surfacing mechanisms and optical (or other) sensors configured to monitor the patient's movements (e.g., how the patient is moving and/or breathing.

The AI model **111** may be stored in the system database **114b**. The AI model **111** may be trained using data received/retrieved from the electronic data sources **120** and may be executed using data received from the end-user devices, the medical device **160,** and/or the sensor **163**. In some embodiments, the AI model **111** may reside within a data repository local or specific to a clinic. In various embodiments, the AI model **111** may use one or more deep learning engines to develop a treatment plan for a patient using radiation therapy. For instance, the analytics server **114a** may transmit patient attributes from the sensor **163** and execute the AI model **111** accordingly. The analytics server **114a** may then display the results on one or more end-user devices **140**. In some embodiments, the analytics server **114a** may change one or more configurations of the medical device **160** based on the results predicted by the AI model **111**.

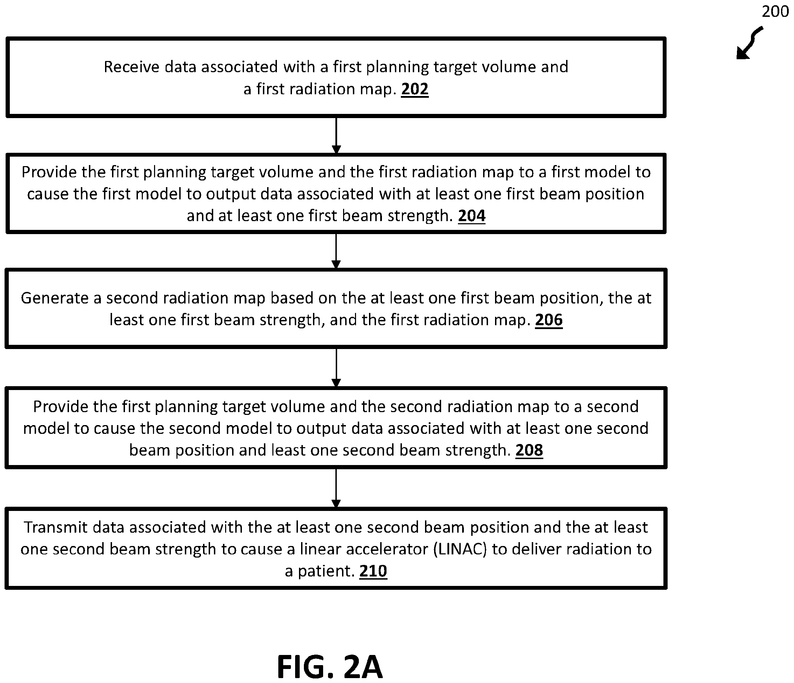

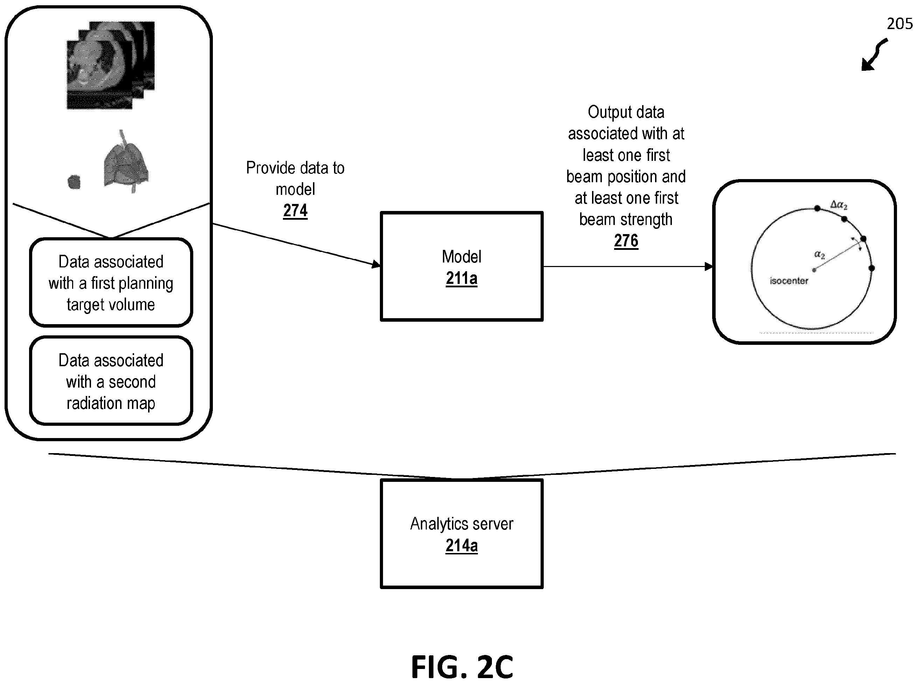

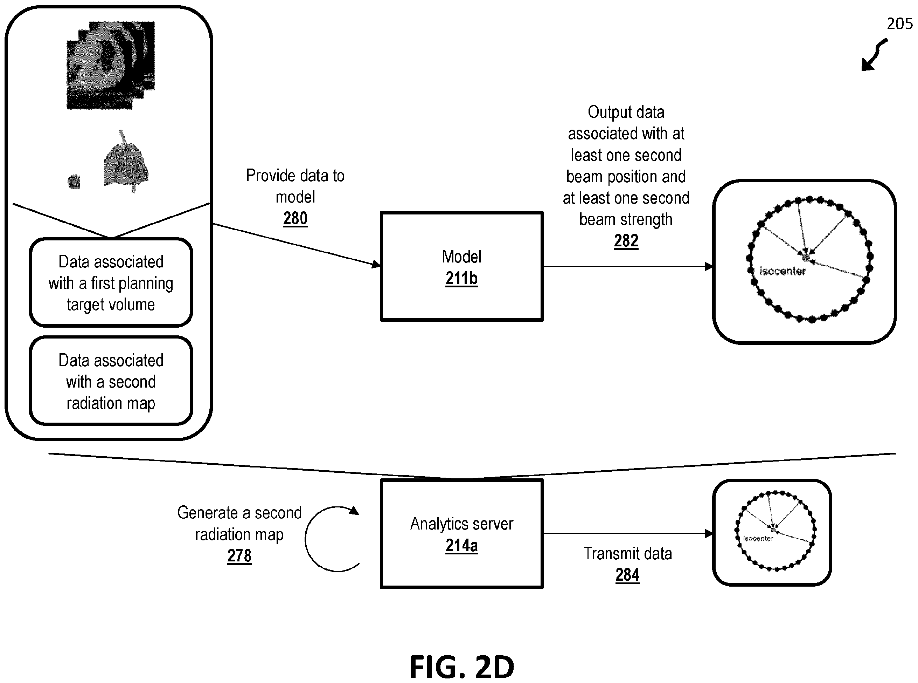

Referring to **FIG. 2A,** illustrated is a diagram of process **200** for planning radiation therapy, in accordance with an embodiment. The process **200** may include operations **202-210**. However, other embodiments may include additional or alternative operations or may omit one or more operations altogether. The process **200** is described as being executed by an analytics server that may be the same as, or similar to, the analytics server **114a** of **FIG. 1.** However, one or more steps of process **200** may be executed (completely, partially, and/or the like) by any number of computing devices operating in the distributed computing system described in **FIG. 1.** For instance, one or more computing devices may perform part, or all of the operations described in **FIG. 2A.**

At operation **202,** an analytics server receives data associated with a first planning target volume and a first radiation map. For example, first planning target volume may represent one or more areas within a body of a patient that are identified (e.g., by a clinician such as an oncologist and/or the like) that are to receive energy (e.g., energy associated with radiotherapy treatment such as high-energy X-rays and/or the like). Additionally, the first planning target volume may be associated with a target mask. The target mask may represent a portion of one or more areas within the body of the patient that are to receive energy during treatment of the patient. In some embodiments, the first radiation map may represent one or more amounts of energy to be delivered to one or more areas within the first planning target volume. The one or more amounts of energy may be measured as counts per second, microsieverts per hour, and/or the like. In some embodiments, the first planning target volume and the first radiation map may be associated resolutions corresponding to the first scale. As will be described below, the resolutions at the first scale may be lower (e.g., coarser) than the resolutions at subsequent scales.

At operation **204,** the analytics server provides the first planning target volume and the first radiation map to a first model to cause the first model to output data associated with at least one first beam position and at least one first beam strength. For example, the analytics server may provide the first planning target volume and the first radiation map to the first model, where the first model is trained to output the at least one first beam position and the at least one first beam strength. In some embodiments, the first beam position corresponds to one or more devices (e.g., energy delivery devices such as electron guns and/or waveguides) associated with a medical device (e.g., a medical device that is the same as, or similar to, medical device **160** such as a LINAC). In some embodiments, the first beam position may correspond to one or more beam angles. The one or more beam angles may be angular positions relative to an axis along which one or more energy delivery devices are configured to transmit energy when delivering energy to the tissue of the patient. It will be understood that this axis is generally positioned parallel during procedures where the medical device is delivering energy to the tissue of the patient, and points along this axis may be referred to as an isocenter. Additionally, or alternatively, the one or more beam angles may be angular positions relative to a planning target volume of a patient.

In some embodiments, the one or more beam angles may be finite (e.g., correspond to certain predetermined angles relative to the axis and/or the planning target volume of the patient) as represented within a candidate angle space. Additionally, or alternatively, the one or more beam angles may correspond to ranges of angles. In some embodiments, the one or more beam angles may correspond to one or more scales. For example, at a first scale, the one or more beam angles may include eleven beam angles, where each beam angle corresponds to positions about the axis that are spaced approximately 33 degrees apart. In an example, at a second scale, the one or more beam angles may include sixteen beam angles, where each beam angel corresponds to positions about the axis that are spaced approximately 22.5 degrees apart. In examples, at a third scale, the one or more beam angles may include thirty-two beam angles, where each beam angle corresponds to positions about the axis that are spaced approximately 11.25 degrees apart. It will be understood that, in examples, the first scale maybe coarser (e.g., offer less granular selection when selecting one or more beam angles at which energy is to be delivered) than the second scale, and the second scale coarser than the third.

In yet another illustrative example, at the first scale, angle candidates may include 0, 30, 60, 90, 120, 150, 180, 210, 240, 270, 300, and 330 degrees (with an interval of 30 degrees between angles). In this example, at the second scale, angle candidates may include 0, 15, 30, 45, 60,75, 90, 105, 120, 135, 150, 165, 180, 195, 210, 225, 240, 255, 270, 285, 300, 315, 330, and 345 degrees (with an interval of 15 degrees between angles). It will be understood that certain angles within the candidate angle space (e.g., 0, 30, 60 degrees, etc.) may correspond to both the first scale and the second scale. And in the above illustrative example, the angles at the first scale are included as a subset of angles in the second scale. It will be understood that, with respect to IMRT procedures, angles can be selected within the candidate angle space; and with respect to VMAT procedures, start and end points may be associated with the start and end of a particular interval corresponding to a scale that is associated with the candidate angle space.

In some embodiments, the first model is configured to provide data associated with at least one first beam position and at least one first beam strength as an output at a first scale (e.g., as described above). In some embodiments, the first scale is defined by a first range of angles formed within a candidate angle space (e.g., as represented by an angle formed when extending two adjacent beams from a centroid outward within the candidate angle space). The candidate angle space may include one or more positions at which beams may be formed corresponding to positions at which the LINAC may be configured to transmit energy along. In one illustrative example, and again with reference to the first scale, the first range of angles may be formed within the candidate angle space within which the LINAC can be configured to deliver energy. The first scale may further be associated with an angle which, in this illustrative example, may be approximately 99 degrees as measured between a first beam (extending from a point along the axis, referred to as the isocenter, to a first point along an axis orbiting the isocenter about which the LINAC can rotate when delivering energy) and a second beam (extending from the isocenter to a second point along the axis orbiting the isocenter). It will be understood that the first range of angles may be associated with different scales corresponding to different angles.

At operation **206,** the analytics server generates a second radiation map based on the at least one first beam position, the at least one first beam strength, and the first radiation map. For example, the analytics server may generate the second radiation map based on the analytics server determining (e.g., simulating) radiation delivered to the patient based on the at least one first beam position and the at least one first beam strength. In examples, the analytics server may generate the second radiation map based on the analytics server comparing the radiation delivered to the patient based on the at least one first beam position and the at least one first beam strength to the planning target volume. Based on comparing the radiation delivered to the patient based on the at least one first beam position and the at least one first beam strength to the planning target volume, the analytics server may determine the second radiation map, where the second radiation map represents one or more areas within the planning target volume that have not received a sufficient amount of energy. In some embodiments, the second radiation map may be associated a resolution corresponding to the second scale. For example, the second radiation map may be associated with a resolution that is greater than the resolution associated with the first radiation map. In this way, the analytics server may determine beam angles and beam strengths at increasingly finer scales, enabling more precise beam placements and power level selection at each scale.

In some embodiments, the analytics server repeats the functions performed with respect to operations **204** and **206** iteratively until converging on an optimized first beam position and an optimized first beam strength. For example, the analytics server may iteratively provide the first planning target volume and successive versions of the second radiation map to the model to cause the model to output successive versions of the least one first beam position and at least one first beam strength. The analytics server may repeat these operations until the least one first beam position and at least one first beam strength that are output by the model converge.

At operation **208,** the analytics server provides the first planning target volume and the second radiation map to a second model to cause the second model to output data associated with at least one second beam position and at least one second beam strength. For example, the analytics server may provide the first planning target volume and the second radiation map to the second model, where the second model is trained to output the at least one second beam position and the at least one second beam strength. In this example, the at least one second beam position corresponds to the at least one second beam strength. In some embodiments, the at least one second beam position corresponds to one or more devices associated with a medical device as described herein. In some embodiments, the second beam position may correspond to one or more beam angles at which a medical device is configured to transmit energy. In some embodiments, with respect to the second scale, the first planning target volume and the second radiation map may be associated with a resolution corresponding to the second scale. For example, the first planning target volume and the second radiation map may be associated with a resolution that is greater (e.g., higher) than the resolution of the first planning target volume and the first radiation map analyzed by the analytics server at the first scale. By virtue of the increasing resolution at each scale, the analytics server may be able to achieve robust optimization of the beam position and beam strength as well as precision with respect to the position at which the beam is delivered to the planning target volume.

In some embodiments, the at least one second beam position may correspond to a position corresponding to a second scale different from positions corresponding to the first scale. For example, the second scale at which the one or more second beam angles are output by the second model may be finer (e.g., offer greater granular selection when selecting one or more beam angles at which energy is to be delivered) than the scale at which the one or more first beam angels are output by the first model. In some embodiments, the second scale may be associated with a second set of candidate angles within the candidate angle space. For example, the second scale may be associated with an angle that is formed at the second scale (e.g., as measured by projecting adjacent beams from a centroid outward), where the angle is smaller than an angle formed by two adjacent beams at the first scale (described above). In this way, the second model may be configured to provide as output the at least one second beam position, where the at least one beam position may be positioned at more precise locations when compared to the at least one beam position that can be provided as output by the first model.

In some embodiments, the second model is configured to provide data associated with the at least one second beam position and the at least one second beam strength as an output at a second scale (e.g., as described above). In some embodiments, the second scale is associated with a second set of candidate angles formed within the candidate angle space. In one illustrative example, where an angle is formed by two adjacent beams at the first scale, a similar angle formed between two adjacent beams at the second scale may be smaller, resulting in a closer grouping of possible beam angles at the second scale. In this way, a second beam may be transmitted at a position that is within the first range but which the first model could not have provided as an output, enabling the second model to identify the second beam position from among one or more beam positions that are within a restricted search space associated with the first scale, thereby focusing the output of the second model.

In some embodiments, the analytics server generates a third radiation map based on the at least one second beam position, the at least one second beam strength, and the second radiation map. For example, the analytics server may generate the third radiation map based on the analytics server determining (e.g., simulating) radiation delivered to the patient based on the at least one second beam position and the at least one second beam strength. In examples, the analytics server may generate the third radiation map based on the analytics server comparing the radiation delivered to the patient based on the at least one second beam position and the at least one second beam strength to the planning target volume. Based on comparing the radiation delivered to the patient based on the at least one second beam position and the at least one second beam strength to the planning target volume, the analytics server may determine the third radiation map, where the third radiation map represents one or more areas within the planning target volume that have not received a sufficient amount of energy.

In some embodiments, the analytics server repeats the functions performed with respect to operation 208 iteratively until converging on an optimized second beam position and an optimized second beam strength. For example, the analytics server may iteratively provide the first planning target volume and successive versions of the third radiation map to the model to cause the model to output successive versions of the least one second beam position and at least one second beam strength. The analytics server may repeat these operations until the least one second beam position and at least one second beam strength that are output by the model converge.

At operation **210,** the analytics server transmits data associated with the at least one second beam position and the at least one second beam strength to cause a medical device to deliver energy to a patient. For example, the analytics server may transmit the data associated with the at least one second beam position and the at least one second beam strength to cause the medical device to deliver radiation to a patient, the data configured to cause the medical device to operate the energy delivery device in accordance with the at least one second beam position and the at least one second beam strength. In some embodiments, the analytics server may update the data associated with the at least one second beam position and the at least one second beam strength to include the at least one first beam position and the at least one first beam strength. The analytics server may then transmit the data associated with (1) the at least one first beam position and the at least one first beam strength, and (2) the at least one second beam position and the at least one second beam strength to cause the medical device to operate the energy delivery device.

*Training models at different scales*

In some embodiments, the one or more models described with respect to processes 200 may be trained. For example, the one or more models (e.g., the first model and the second model) may be trained based on reinforcement learning-based techniques such as model-free approaches that rely on sampling and world-exploration (e.g., gradient policy optimization, deep Q-learning, and/or the like) as well as model-based approaches that incorporate a world-model for increased efficiency in exploration and learning. In an example, where one or more agents are associated with corresponding training models (e.g., deep neural networks (DNNs) and/or the like, which are sometimes referred to as "policies"), the analytics server may train the training models by initializing and/or updating weights of the training models and test the training models using an environment. Based on simulated outputs of the environment corresponding to the training models, the agents may then update the corresponding training models by updating one or more weights of the corresponding training models, to cause the training models to make more accurate predictions when tested. Training using reinforcement learning-based techniques will be described in greater detail below.

During training, the analytics server may first obtain (e.g., receive) a dataset (which can be referred to as a parametric model $\beta$. The dataset may represent (e.g., $\beta$ may contain) data associated with a set of patients (N patients), each patient being associated with one or more computed tomography (CT) scans, organs at risk (OARs), planning target volumes (PTVs), and radiation maps (sometimes referred to as dose prescriptions). In this example, the dataset may represent data associated with patients that were previously treated using a medical device as described herein. Additionally, the analytics server may obtain a dose prediction model configured to receive as input the CT scans, OARs, and/or PTVs, and provide, as an output, the radiation maps.

The analytics server may next initialize an experience queue ("Q") for each agent of the one or more agents. Additionally, or alternatively, the analytics server may initialize an experience queue that is shared across one or more agents. In some embodiments, the experience queue represents transitions associated with one or more training models ("$\theta$") that are associated with one or more scales (e.g., a set of scales representing M levels of scale). In some embodiments, each scale of the one or more scales may be associated with one or more beam positions corresponding to a range of beam positions and/or one or more beam strengths corresponding to a range of beam strengths.

In some embodiments, the analytics server constructs a scale space for each of the one or more scales which may be used to separate the transitions in the experience queue. For example, the analytics server may construct a scale space comprising a first scale, a second scale, and a third scale. It will be understood that more, or fewer, scale spaces may be constructed as desired. In some embodiments, as one or more agents generate one or more sample experience episodes (described below) the sample experience episodes may be associated with (e.g., separated by) one or more scales as described herein.

In some embodiments, the analytics server may initialize a plurality of sample experience episodes. For example, the analytics server may initialize a plurality of sample experience episodes, where one or more sample experience episodes corresponds to each agent of the one or more agents and/or one or more scales of the scale space. During each sample experience episode, the analytics server may provide data associated with an individual patient of the set of patients to an agent. The agent may then provide the data associated with an individual patient to the training model associated with the agent to cause the training model to provide an output. The output may include one or more beam positions and one or more beam strengths that are expected to target tissue in the PTVs of the patient.

In some embodiments, the agent provides the one or more beam positions and one or more beam strengths to a simulator that generates a simulated output. For example, the agent may provide the one or more beam positions and one or more beam strengths to a simulator that generates a simulated output representing the delivery of energy to tissue of the patient by a medical device (e.g., a LINAC). In some embodiments, the simulated output is compared to goal radiation map (sometimes referred to as a "dose prescription") to determine a reward. For example, the simulated output may be compared to the goal radiation map to determine a degree to which the simulated output corresponds to the goal radiation map, the degree representing the reward. In some embodiments, the analytics server may store data associated with each experience episode (e.g., the training model involved, the weights of the training model involved, the data associated with the individual patient involved, one or more transitions in the weights of the training model involved during the experience episode, one or more rewards associated with the one or more transitions, and/or the like) in the experience queue.

In some embodiments, the analytics server may sample one or more parameters of the one or more training models that are represented in the experience queue to optimize a set of models. For example, analytics server may sample one or more parameters of the one or more training models that are represented in the experience queue to determine sets of parameters (e.g., weights) for models to be used during inference. In this example, each model of the set of models may correspond to a respective scale of the one or more scales in the scale space. In some embodiments, the analytics server may sample the one or more parameters of the one or more training models to determine a set of models that are associated with an optimized set of parameters based on one or more transitions. For example, where a first training model is associated with a first transition having a first reward and a second training model is associated with a second transition having a second reward that is less than the first reward, the analytics server may determine a model (e.g., one or more weights of the model) based on one or more weights of the first training model. In this way, the analytics server may select parameters for each model of the set of models that optimize the output of each model. In some embodiments, once the model is optimized (e.g., converges such that performance of the model stabilizes), the analytics server an output the model. In some embodiments, the analytics server may sample the one or more parameters of the one or more training models that are represented in the experience queue to optimize a set of models, where the set of models includes a model associate with each scale.

During training, the analytics server restricts exploration that can be performed by each agent. For example, the analytics server may restrict exploration that can be performed by each agent to one or more scale spaces. In an example, the analytics server may restrict exploration when generating a model at a given scale space by sampling experience episodes at a given scale when optimizing the model at the given scale space. One illustrative restriction can include restricting the positions at which one or more beams may be generated by the medical device based on a given scale space.

By virtue of the training techniques described herein, the models described can be trained so as to incorporate one or more preferences and/or one or more machine-specific requirements. For example, where the goal radiation maps are generated by clinicians, the models may be generated by the analytics server such that they approximate the goal radiation maps. Additionally, or alternatively, during simulation, the simulated output may be generated based on one or more machine-specific requirements. These can include, for example, a minimum angle that is required between two adjacent beam angles, minimum or maximum beam strengths that can be delivered, and/or the like. In this way, the models output by the analytics server during training can capture specific clinician requirements at inference time in a number of different possible ways, such as by leveraging the stochasticity of the transition dynamics to generate different solutions to chose from and constraining the inference to certain spaces of the parameter space, ensuring that the solution is aligned with given requirements of medical device for which the models may be used with when delivering energy to patients.

And by virtue of the implementation of models (e.g., sets of models configured to generate outputs at different scales) as described herein, the amount of time and/or resources needed to perform inferences using the models can be reduced. This, in turn, can result in faster generation of beam positions and corresponding beam strengths that are used to develop treatment plans. Additionally, by restricting the space in which each model can cause a medical device to operate within (e.g., beam positions at each scale space) the computing resources needed to generate such beam positions and corresponding beam strengths can be reduced, enabling faster analysis of data associated with individuals by systems that are the same as, or similar to, the analytics server described herein.

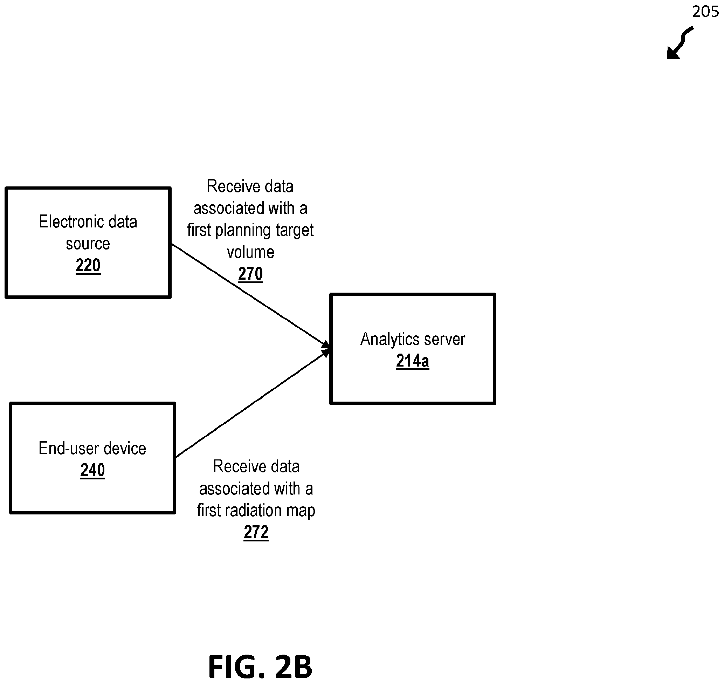

Referring now to **FIGS. 2B-2D,** illustrated is a flowchart of an implementation **205** relating to a process for planning and delivering radiation therapy. As illustrated in **FIGS. 2B-2D,** implementation **205** includes an electronic data source **220** (which may be the same as, or similar to, electronic data source **120** of **FIG. 1**), an end-user device **240** (which may be the same as, or similar to, the end user device **140** of **FIG. 1),** and an analytics server **214a** (which may be the same as, or similar to, the analytics server **114a** of **FIG. 1).**

At operation **270,** the analytics server **214a** receives data associated with a first planning target volume from the electronic data source **220.** At operation **272,** the analytics server **214a** receives data associated with a first radiation map from the end user device **240.** In some implementations, the data associated with the first radiation map may include data associated with one or more user inputs that are configured to cause the analytics server **214a** to generate the first radiation map. More specifically, the one or more user inputs may correspond to one or more commands provided by the user to define the area of a PTV of a patient. In these implementations, the analytics server **214a** may generate the first radiation map based on one or more CT scans (or other similar scans) and/or one or more treatment plans represented by the one or more user inputs.

At operation **274,** the analytics server **214a** provides data to a model. For example, the analytics server **214a** may provide the data associated with the first planning target volume and the data associated with the first radiation map to the model **211a** to cause the model **211a** to generate an output. In this example, the model **211a** may be trained to provide outputs at a first scale, as described herein. At operation **276,** the analytics server **214a** causes the model **211a** to output data associated with at least one first beam position and at least one first beam strength. In some implementations, the data associated with at least one first beam position and at least one first beam strength may represent one or more beams extending from an isocenter outward (toward a device that is emitting energy such as a collimator of a LINAC).

At operation **278,** the analytics server **214a** generates a second radiation map. At operation **280** the analytics server **214a** provides the data associated with the first planning volume and the data associated with the second radiation map to a second model **211b.** In this example, the model **211b** may be trained to provide outputs at a second scale, as described herein. At operation **282,** the analytics server **214a** causes the model **211b** to output data associated with at least one second beam position and at least one second beam strength. In some implementations, the data associated with at least one second beam position and at least one second beam strength may represent one or more beams extending from an isocenter outward (toward a device that is emitting energy such as a collimator of a LINAC).

At operation **284,** the analytics server **214a** transmits data associated with the at least one second beam position and at least one second beam strength. For example, the analytics server **214a** transmits data associated with the at least one second beam position and at least one second beam strength to a medical device computer (e.g., a medical device computer that is the same as, or similar to, medical device computer **162)** to enable the medical device computer to control a medical device (e.g., a medical device that is the same as, or similar to, medical device **160** of **FIG. 1**) when delivering energy to a patient.

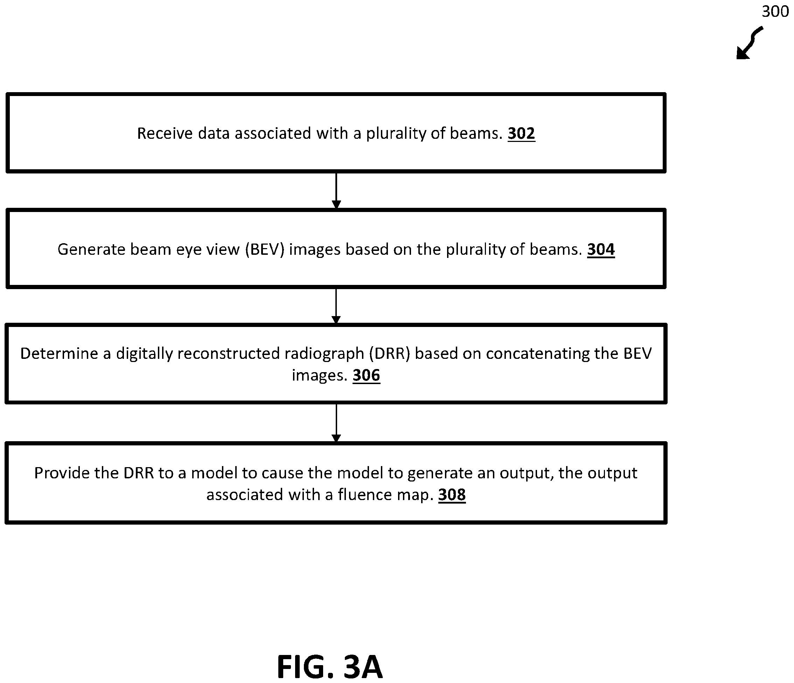

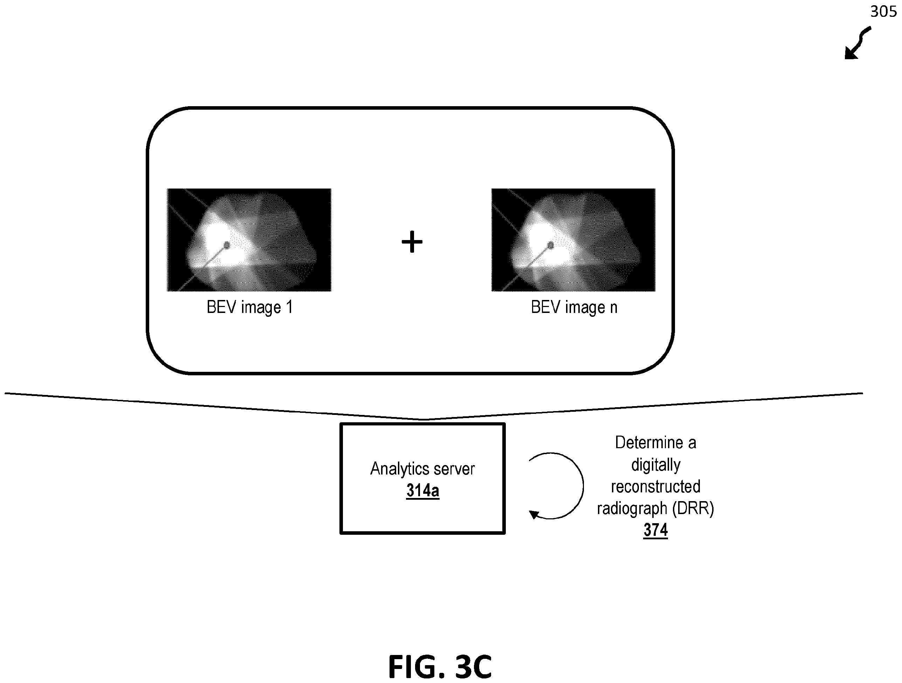

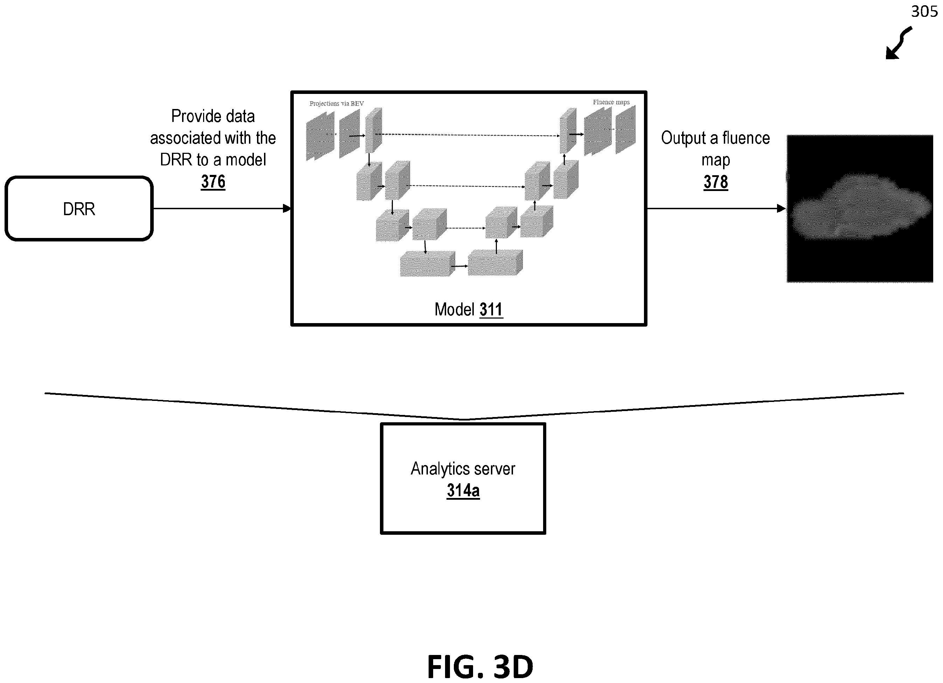

Referring to **FIG. 3A,** illustrated is a diagram of process **300** for generating fluence maps when planning radiation therapy, in accordance with an embodiment. The process **300** may include operations **302-308.** However, other embodiments may include additional or alternative operations or may omit one or more operations altogether. The process **300** is described as being executed by an analytics server that may be the same as, or similar to, the analytics server **114a** of **FIG. 1.** However, one or more steps of process **300** may be executed (completely, partially, and/or the like) by any number of computing devices operating in the distributed computing system described in **FIG. 1.** For instance, one or more computing devices may perform part, or all of the operations described in **FIG. 3A.**

At operation **302,** the analytics server receives data associated with a plurality of beams. For example, the analytics server may receive data associated with a plurality of beams that may be generated by one or more devices (e.g., electron guns and/or waveguides) associated with a medical device (e.g., a medical device that is the same as, or similar to, the medical device **160** of **FIG. 1).** While reference will be made to medical device that are the same as, or similar to, LINACs, it will be understood that the techniques described herein may be implemented using any suitable medical device capable of delivering energy to at least a portion of a patient as described herein. In some embodiments, the plurality of beams is associated with a treatment plan for a patient. For example, the plurality of beams may be specified by a treatment plan generated using, for example, the techniques for planning radiation therapy discussed above with respect to process **200** (see **FIG. 2A).**

In some embodiments, the analytics server receives data associated with a plurality of leaves corresponding to each beam of the plurality of beams. For example, where a LINAC is configured to deliver energy to a patient, the LINAC may be configured to set one or more positions of one or more leaves when generating corresponding beams. In this example, the one or more leaves may be leaves of a collimator of the LINAC that are configured to cause the LINAC to generate a beam in a shape. In some embodiments, the one or more positions of the one or more leaves may represent a configuration of the plurality of leaves when arranged to cause the LINAC to generate the beam in the shape. The configuration may further be associated with a shape for the beam that is intended to be transmitted to the body, the shape targeting at least a portion of the tissue (e.g., tissue associated with a PTV) of the patient. The configuration may be used to generate beam eye view images as described herein.

In some embodiments, the analytics server receives data associated with a plurality of positions of a plurality of leaves, the plurality of positions being further associated with a plurality of time steps. For example, the analytics server may receive data associated with a plurality of positions of a plurality of leaves, with sets of positions at each time step referred to as a sub-configuration. In one illustrative example, in the case where there are a first leaf and a second leaf in a leaf pair, the plurality of positions may represent movement of the first leaf and the second leaf along a common direction, forming a shape that is iteratively moved such that the corresponding beams, when extending through the body of the patient, travels across the PTV. In other examples, it will be understood that there may be multiple leaf pairs and that the leaf pairs may move in synchronization with one another, in different direction from one another, and/or at different rates of speed with one another across the plurality of time steps.

At operation **304,** the analytics server generates BEV images based on the plurality of beams. For example, the analytics server may generate BEV images based on the analytics server receiving and/or determining the data associated with the plurality of beams. For example, the analytics server may generate the BEVs based on the beams (e.g., the axis along which the beams are transmitted relative to a patient and/or a relative position between a point at which the energy is generated by the medical device and a point along the PTV). Additionally, the analytics server may generate the BEVs based on power levels associated with the beams. For example, the analytics server may generate the BEVs based on power levels at which the device of the medical device generating the beams configured to generate power. In some embodiments, the power level at which each beam is generated may be constant. Alternatively, the power level at which each beam is generated by be variable.

In some embodiments, the analytics server generates the BEV images based on a configuration of a plurality of leaves. For example, as noted above, the medical device (e.g., a LINAC) may include a collimator having a plurality of leaves. In this example, the plurality of leaves may be located at positions representing a configuration. The analytics server may then generate the BEV images based on the configuration. In this example, the BEV images may represent the beam as shaped by the plurality of leaves while in the configuration. In some embodiments, the analytics server generates the BEV images based on delivery of energy by the medical device at each time step of a plurality of time steps. For example, where a number of beams are delivered at a number of corresponding time steps, the analytics server may generate the BEV images at each time step of the plurality of time steps.

At operation **306,** the analytics server determines a DRR based on concatenating the BEV images. For example, the analytics server may determine (e.g., generate) the DRR based on concatenating the BEV images generated at a plurality of time steps. In this example, the plurality of time steps may be associated with the treatment plan for the patient. The DRR may represent a multi-channel 2D image that can be provided to a model as described herein. For example, the DRR may include multiple channels, where each channel of the multiple channels corresponds to a BEV image of the set of BEV images that were concatenated by the analytics server.

At operation **308,** the analytics server provides the DRR to a model to cause the model to generate an output, the output associated with a fluence map. In some embodiments, the output of the model may include fluence map predictions for each beam associated with the DRR, where the set of all beams is represented as K beams $\{\hat{y}_i\}_{i=1}^{K}$. The model may also be associated with a loss function defined as $L = \sum_{i=1}^{K} Dist(y_i, \hat{y}_i)$, where the $y_i$ is the referenced fluence map of i-th beam, and $Dist(y_i, \hat{y}_i)$ is the distance between $y_i$ and $\hat{y}_i$. In some embodiments, each $y_i$ could also be a vector in which each element captures a different characteristics of line projection through a 3D dose map (e.g., minimum dose, maximum dose, average dose, and/or the like). The goal of the network in this case is to disentangle the information and compute the prospective fluence map for each bean angle given the total 3D dose. While the above description is described in association with beams generated during an intensity-modulated radiation therapy (IMRT) procedure, in the case of volumetric modulated arc therapy (VMAT), a finite set of angles from an arc could be considered as the one or more beams described and the same analysis performed. In some embodiments, the analytics server may consider interpolation between the discrete angles to compute a continuous arc-based fluence map.

In some embodiments, the analytics server may provide the DRR to the model where the model comprises one or more encoders and one or more decoders. For example, where the model is associated with a U-net architecture, the model may comprise one or more encoders associated with a contracting path and one or more corresponding decoders associated with an expanding path, the one or more encoders and one or more decoders being associated with one or more levels. In this example, the analytics server may provide the DRR to the model by providing individual BEV images to a first encoder at a first level of the model. The first encoder may be configured to receive the individual BEV images and provide two outputs: a first output which is provided by the first encoder to a second encoder along the contracting path, and a second output which is provided to a first decoder which corresponds to the first encoder, the first decoder associated with the expanding path. Depending on the number of layers of the model, the process may be repeated for corresponding encoders and decoders at each successive layer of the model. In some embodiments, at the last (or n-th) layer of the model, the output of the encoder at the n-th layer is provided as the input of the decoder at the n-th layer. The decoder then provides an output to the decoder associated with the layer above the n-th layer, which continues for each successive layer until reaching the first decoder.

In some embodiments, the outputs of each encoder may be provided to one or more convolution layers that are associated with the encoder before being provided to the next encoder along the contracting path of the model or, in the case of the n-th encoder, the n-th decoder along the expanding path of the model. Additionally, or alternatively, the output of each encoder may be provided to a max pooling layer (e.g., after passing through the one or more convolution layers) before being provided to the next encoder along the contracting path of the model. In some embodiments, the output of each decoder along the expanding path of the model may be provided to an up-convolution layer. Additionally, or alternatively, the output of each decoder may be provided to one or more convolution layers (e.g., after passing through the up-convolution layer). In some embodiments, the last decoder of the model may provide as an output the one or more fluence maps. For example, the last decoder of the model may provide as an output the one or more fluence maps where the one or more fluence maps correspond to the one or more BEV images of the DRR that are provided as input to the model.

In some embodiments, the analytics server may generate a leaf motion sequence. For example, the analytics server may generate a leaf motion sequence based on the analytics server generating the one or more fluence maps. In examples, the analytics server may generate the leaf motion sequence based on the one or more fluence maps and confirm whether or not the leaf sequence ultimately achieves a desired dose-volume histogram (e.g., to confirm whether or not the desired amount of energy is delivered to the PTV of the patient).

In some embodiments, the analytics server may train the model. For example, the analytics server may train the model based on one or more BEV images and one or more corresponding fluence maps. In this example, the one or more BEV images and one or more corresponding fluence maps may be generated at an earlier point in time. In some embodiments, the analytics server trains the model based on one or more BEV images and one or more corresponding fluence maps, where the fluence maps represent measurements of energy delivered to a patient during treatment of the patient.

In some embodiments, the analytics server trains the model based on the analytics server providing the one or more BEV images to the model to cause the model to provide an output, the output representing a fluence map. The analytics server may then compare the output representing the fluence map to the corresponding fluence map (e.g., the fluence map generated at the earlier point in time corresponding to the input BEV image). In this example, the analytics server may determine a difference between the output representing the fluence map and the corresponding fluence map (generated earlier in time) and the analytics server may update the layers of the model. In some embodiments, the analytics server may update the layers of the model using one or more backpropagation techniques (e.g., stochastic gradient descent, root mean square propagation, and/or the like).

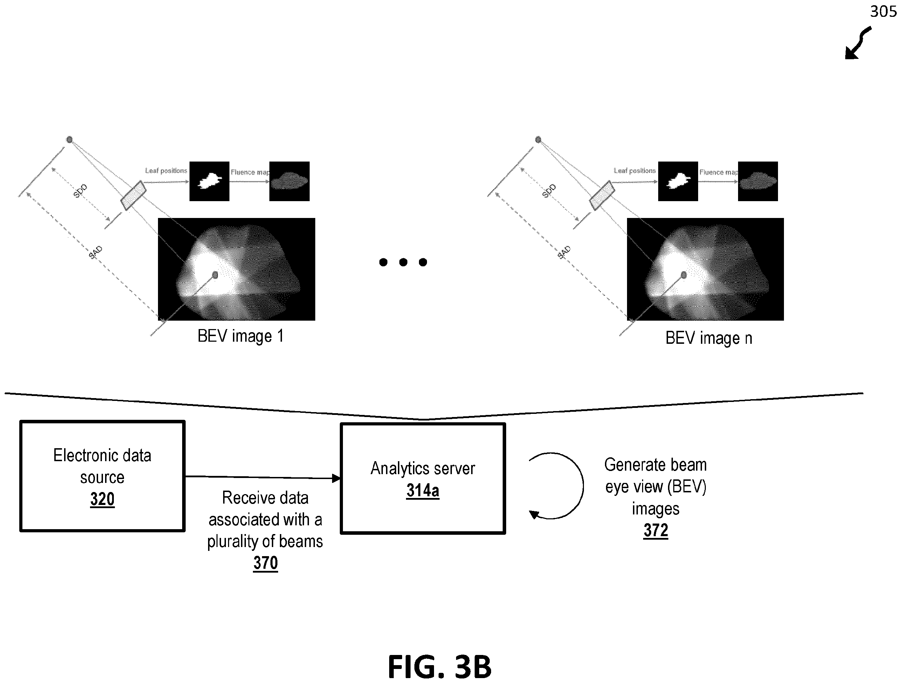

Referring now to **FIGS. 3B-3D,** illustrated is a flowchart of an implementation **305** relating to a process for generating fluence maps when planning radiation therapy. As illustrated in **FIGS. 3B-3D,** implementation **305** includes an electronic data source **320** (which may be the same as, or similar to, electronic data source **120** of **FIG. 1)** and an analytics server **314a** (which may be the same as, or similar to, the analytics server **114a** of **FIG. 1).**

At operation **370** the analytics server **314a** receives data associated with a plurality of beams. In some implementations, the analytics server **314a** may receive data associated with a plurality of beams to be generated and transmitted by a medical device (e.g., a medical device that is the same as, or similar to, the medical device **160** of **FIG. 1,** such as a LINAC). At operation **372,** the analytics server **314a** generates BEV images. In some implementations, the analytics server **314a** generates the BEV images based on the data associated with the plurality of beams. Additionally, the analytics server **314a** may generate the BEV images based on a predictive geometry associated with the medical device (e.g., based on dosimetric models such as analytic models, Monte Carlo simulations, deterministic models, and/or the like).

At operation **374,** the analytics server **314a** determines a DRR. In some implementations, the analytics server **314a** determines the DRR based on the BEV images. For example, the analytics server **314a** may determine the DRR based on the analytics server concatenating the BEV images to generate the DRR.

At operation **376,** the analytics server **314a** provides data associated with the DRR to a model **311.** In some implementations, the analytics server **314a** provides the data associated with the DRR to the model **311,** where the model **311** is associated with a U-net architecture. In examples, the model **311** may be trained as described herein to output a fluence map. At operation **378,** the analytics server **314a** causes the model **311** to output the fluence map.

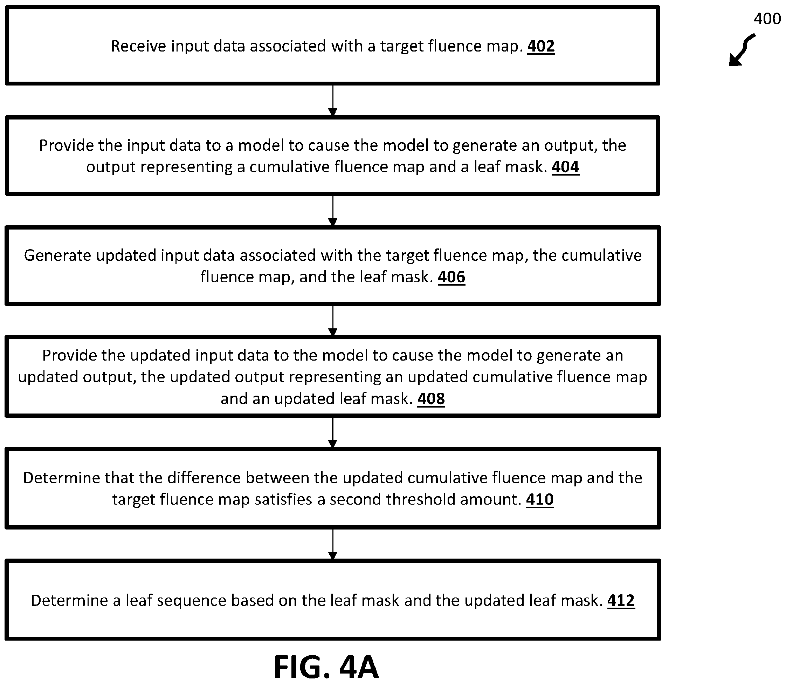

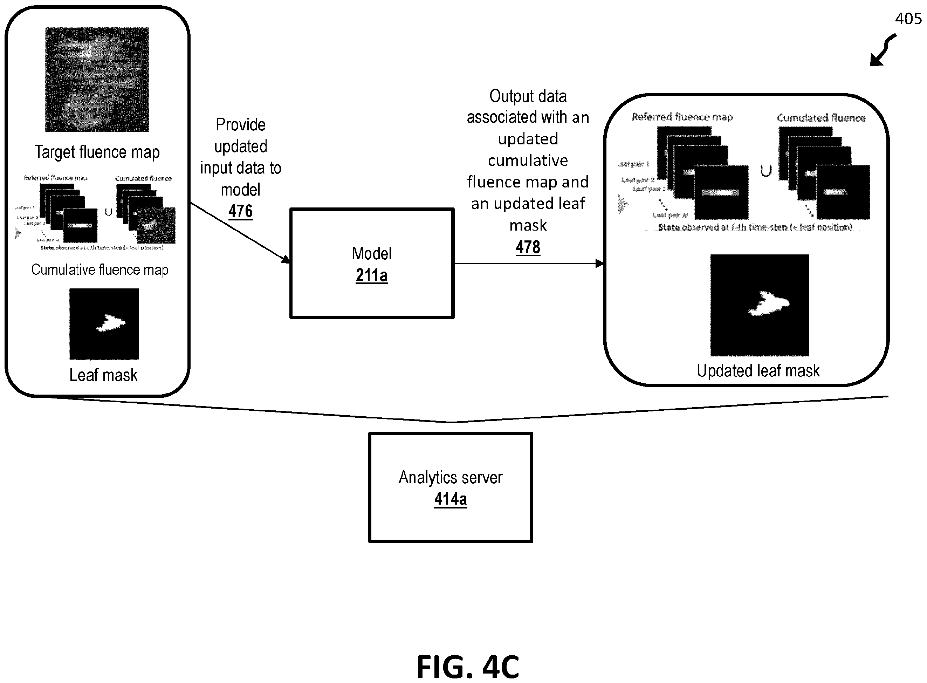

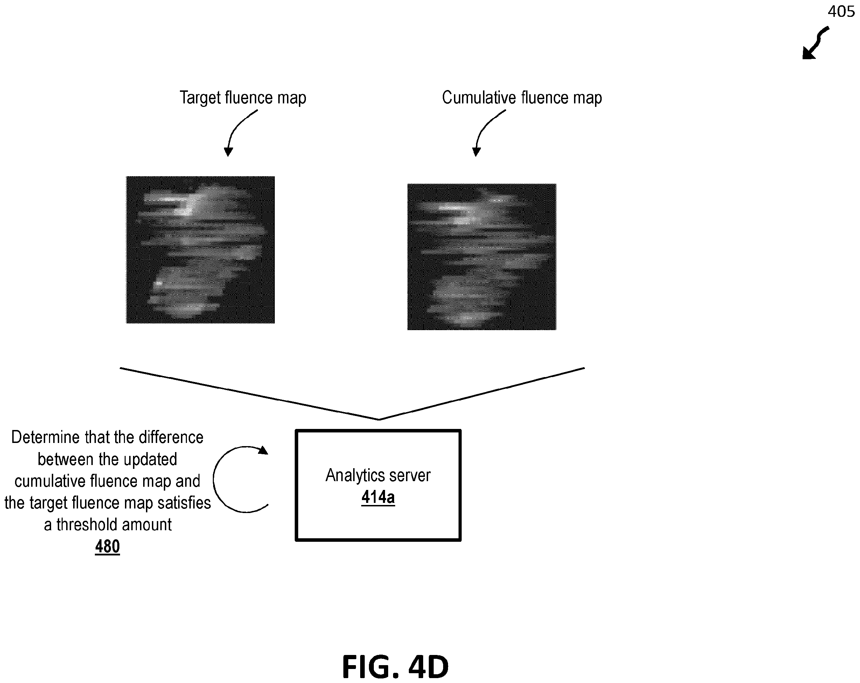

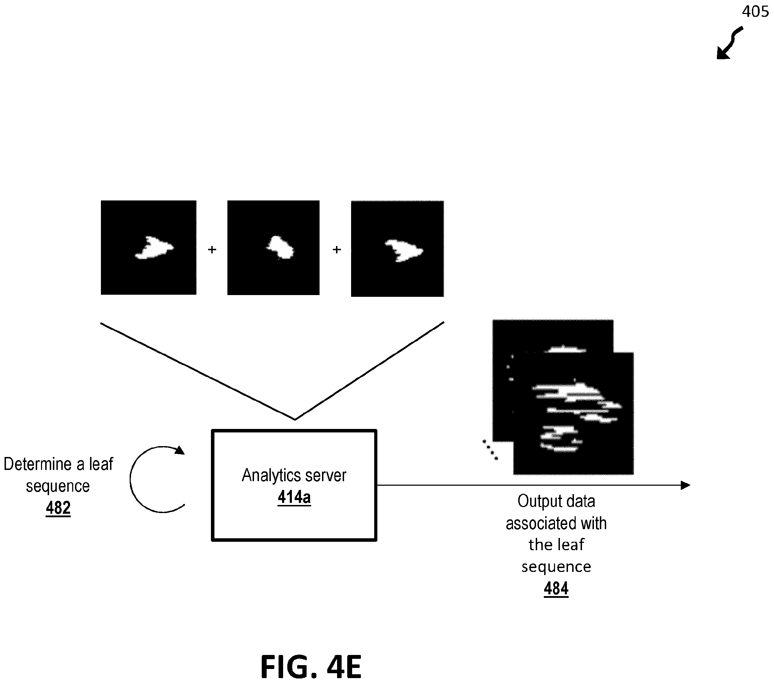

Referring to **FIG. 4A,** illustrated is a diagram of process **400** for generating fluence maps when planning radiation therapy, in accordance with an embodiment. The process **400** may include operations **402-412.** However, other embodiments may include additional or alternative operations or may omit one or more operations altogether. The process **300** is described as being executed by an analytics server that may be the same as, or similar to, the analytics server **114a** of **FIG. 1.** However, one or more steps of process **300** may be executed (completely, partially, and/or the like) by any number of computing devices operating in the distributed computing system described in **FIG. 1.** For instance, one or more computing devices may perform part, or all of the operations described in **FIG. 3A.**

At operation **402,** the analytics server receives input data associated with a target fluence map. For example, the analytics server may receive input data associated with a target fluence map, where the target fluence map represents a goal amount of energy to deliver to a patient. In this example, the goal amount of energy to deliver to the patient may be associated with energy delivery via a medical device (e.g., a medical device that is the same as, or similar to, medical device **160** of **FIG. 1)** such as, for example, a LINAC. In some embodiments, the goal amount of energy may be represented as intensities of energy (e.g., radiation) to be delivered per unit area of tissue of a patient (e.g., per unit area of a PTV). In some embodiments, the target fluence map corresponds to an angle at which a PTV may be viewed.

In some embodiments, the target fluence map is represented as a vector image. For example, the analytics server may receive a target fluence map which is represented as a vector image. Additionally, or alternatively, the analytics server may receive a target fluence map which is represented as a two dimensional image (e.g., a rasterized image). In this example, the analytics server may update the target fluence map such that the target fluence map is a vector image that represents (e.g., closely approximates) the two dimensional image. In this way, the operations discussed herein can be performed based on a smaller state space (e.g., with target fluence maps and cumulative fluence maps described herein which may be, for example, 300 times smaller than when represented as two dimensional images), thereby saving significant computing resources at the inference and training stages described herein.

At operation **404,** the analytics server provides the input data to a model to cause the model to generate an output, the output representing a cumulative fluence map and a leaf mask. In some embodiments, the analytics server may provide the input data to a model to cause the model to generate an output, where the model comprises one or more second models. For example, the analytics server may provide the input data to a model comprising one or more second models, where each of the one or more second models is associated with a leaf pair of the medical device. In this example, the input may be provided to the one or more second models to cause the one or more second models to generate respective outputs, the respective outputs representing one or more leaf masks and cumulative fluence maps. It will be understood that the one or more second models may be configured to receive the input data and provide, as their output, data associate with fluence maps and/or leaf masks corresponding to areas that can be addressed by the corresponding leaf pairs. The model and the one or more second models may be trained as described herein.

In some embodiments, when the input data is initially provided as the input to the model, the input data may also be associated with a cumulative fluence and a current leaf mask. For example, the input data may also be associated with a cumulative fluence and a current leaf mask, where the cumulative fluence is equal to a zero or null value, and where the current leaf mask is associate with one or more initial positions for each leaf of the pairs of leaves of a collimator that can be adjusted during operation of a LINAC. As will be described below, the cumulative fluence and the current leaf mask can be iteratively updated at each time step when planning treatment of a patient.

At operation **406,** the analytics server generates updated input data associated with the target fluence map, the cumulative fluence map, and the leaf mask. For example, the analytics server may generate updated input data associated with the target fluence map, the cumulative fluence map, and the leaf mask based on the analytics server determining a difference between the cumulative fluence map and the target fluence map. In this example, the analytics server may determine the difference between the cumulative fluence map and the target fluence map at each time step when planning treatment of the patient. In some embodiments, the analytics server may determine the difference between the cumulative fluence map and the target fluence map based on the analytics server comparing the intensity values of energy per unit area of the target fluence map and an intensity value of energy per unit area of the cumulative fluence map. The analytics server may then determine whether a difference between one or more of the intensity values of the cumulative fluence map satisfies a first threshold amount when compared to the one or more intensity values of the target fluence map. In examples, the first threshold amount may be a value associated with a difference of the aggregated intensity values of the cumulative fluence map and the aggregated intensity values of the target fluence map. Additionally, or alternatively, the first threshold amount may be a value associated with a difference in one or more intensity values of the cumulative fluence map and one or more corresponding intensity values of the target fluence map.

At operation **408,** the analytics server provides the updated input data to the model to cause the model to generate an updated output, the updated output representing an updated cumulative fluence map and an updated leaf mask. For example, the analytics server may provide the updated input data to the model to cause the model to generate the updated output based on the analytics server updating the input data. In some embodiments, the analytics server provides the updated input data to the model to cause the model to generate a second updated output, the second updated output representing a second updated cumulative fluence map and a second updated leaf mask. In some embodiments, the analytics server may provide the updated input data to a model to cause the model to generate an output, where the model is the same as, or similar to, the model described above (e.g., with respect to operation **404).**

At operation **410,** the analytics server determines that the difference between the updated cumulative fluence map and the target fluence map satisfies a second threshold amount. For example, the analytics server may determine the difference between the updated cumulative fluence map and the target fluence map based on the analytics server comparing the intensity values of energy per unit area of the updated cumulative fluence map and an intensity value of energy per unit area of the target fluence map. The analytics server may then determine whether a difference between one or more of the intensity values of the updated cumulative fluence map satisfies a second threshold amount when compared to the one or more intensity values of the target fluence map. In examples, the second threshold amount may be a value associated with a difference of the aggregated intensity values of the updated cumulative fluence map and the aggregated intensity values of the target fluence map. Additionally, or alternatively, the second threshold amount may be a value associated with a difference in one or more intensity values of the updated cumulative fluence map and one or more intensity values of the target fluence map. In some embodiments, the second threshold amount may less than the first threshold amount. In this way, the analytics server can determine whether the energy delivered to the PTV satisfies the treatment plan for the patient.

In some embodiments, the analytics server repeats operations **404-410.** More specifically, the analytics server may repeat operations **404-410** where, at operation **410,** the difference in one or more intensity values of the updated cumulative fluence map and one or more intensity values of the target fluence map satisfies the first threshold amount but not the second threshold amount. In this way, the analytics server may iteratively provide data to the model and generate output data that can be used to generate a leaf sequence that results in the delivery of energy during treatment of the patient satisfying the treatment plan for the patient.

At operation **412,** the analytics server determines a leaf sequence based on the leaf mask and the updated leaf mask. For example, the analytics server may determine a leaf sequence based on the analytics server determining a difference between the leaf mask and the updated leaf mask. In this example, the analytics server may determine the leaf sequence based on the difference between the leaf mask and the updated leaf mask. In some embodiments, where operations 404-410 are iteratively repeated to address a larger PTV, the leaf mask and the updated leaf masks may be concatenated. In this case, the analytics server may determine the leaf sequence based on the analytics server determining the differences between the iteratively-generated updated leaf masks.

In some embodiments, the analytics server may determine the leaf sequence based on the analytics server determining a first leaf position and a second leaf position associated with a leaf pair of the LINAC. For example, the analytics server may determine the first leaf position and a second leaf position associated with the leaf pair, where the first leaf position and the second leaf position correspond to the leaf mask. In some embodiments, the analytics server may then determine a third leaf position and a fourth leaf position associated with the leaf pair. For example, the analytics server may determine the third leaf position and fourth leaf position associated with the leaf pair, where the third leaf position and the fourth leaf position correspond to the updated leaf mask. The analytics server may then determine a difference between the leaf mask and the updated leaf mask as represented by a change in positions of each leaf of the leaf pair. For example, the analytics server may then determine a difference between the leaf mask and the updated leaf mask as represented by (1) a change in positions of the first leaf position when compared to the third leaf position, and (2) a change in positions of the second leaf position when compared to the fourth leaf position. Similar to above, this may be iteratively performed depending on the number of updated leaf masks generated.

*Training models for the determination of leaf sequences*

In some embodiments, the one or more models described with respect to processes 400 may be trained. For example, the one or more models (e.g., the model and the one or more second models) may be trained based on reinforcement learning-based techniques. Examples of reinforcement learning techniques are described by Schulman et al., "Proximal Policy Optimization Algorithms," available at https://arxiv.org/abs/1707.06347. Given policy net $\pi_\theta$, state $s_t$,

$$r_t(\theta) = \frac{\pi_\theta(a_t|s_t)}{\pi_{\theta\_old}(a_t|s_t)}$$

and action $a_t$, a probability ratio can be denoted as . The main objective may be $L^{clip}(\theta) = \hat{E}[\min(r_t(\theta) \hat{A}_t, clip(r_t(\theta), 1 - \varepsilon, 1 + \varepsilon)\hat{A}_t)]$, where $\hat{A}_t$ is an estimator of the advantage function at timestep t, $\hat{E}[\cdot]$ is the empirical average over a finite batch of samples. An alternative to this clipped surrogate objective is to use a KL divergence as a penalty between the current policy and old policy: $L^{klp}(\theta) = \hat{E}[r_t(\theta)\hat{A}_t - \beta KL(\pi_{\theta_{old}}, \pi_\theta)]$. The value function may be estimated with another network $V_\theta$ (other than policy net), which is trained with a squared-error loss $(V_\theta(s_t) - V_t^{targ})$.

In some embodiments, the one or more models described with respect to process 400 may be trained based on one or more model-free approaches as well as model-based approaches that incorporate a world-model for increased efficiency in exploration and learning. In an example, where one or more agents are associated with corresponding training models (e.g., deep neural networks (DNNs) and/or the like, which are sometimes referred to as "policies"), the one or more agents may each be further assigned to train the corresponding training models based on one or more leaf pairs assigned to the agents. In examples, each leaf pair of a medical device (e.g., a LINAC) may be assigned to an agent from among the one or more agents. In some embodiments, the analytics server may train the training models corresponding to each agent by initializing and/or updating weights of the training models and test the training models using an environment. Based on simulated outputs of the environment corresponding to the training models, the agents may then update the corresponding training models by updating one or more weights of the corresponding training models, to cause the training models to make more accurate predictions when tested. Training using reinforcement learning-based techniques will be described in greater detail below.

During training, the analytics server may first obtain (e.g., receive) a dataset. The dataset may represent data associated with a set of patients (N patients), each patient being associated with one or more computed tomography (CT) scans, organs at risk (OARs), planning target volumes (PTVs), and/or radiation maps (sometimes referred to as dose prescriptions). In this example, the dataset may represent data associated with patients that were previously treated using a medical device as described herein.

The analytics server may next initialize an experience queue ("Q") for each agent of the one or more agents. Additionally, or alternatively, the analytics server may initialize an experience queue that is shared across one or more agents. In some embodiments, the experience queue represents one or more sample experience episodes associated with one or more training models ("$\theta$"), the sample experience episodes representing transitions that can further represent, for example, changes in leaf positions (e.g., a first pair of leaf positions and a second pair of leaf positions). Additionally, the experience queue may represent one or more power levels associated with the transitions at which energy is delivered to a patient while the leaf pair was in the first pair of leaf positions, the second pair of leaf positions, and/or one or more intermediate positions while one or more leaves of the leaf pair moved between the first pair of leaf positions to the second pair of leaf positions.

In some embodiments, the experience queue may represent one or more speeds with which the one or more leaves moved when transitioning from the first pair of leaf positions to the second pair of leaf positions. For example, as the leaf pair moves between the first pair of leaf positions to the second pair of leaf positions, the analytics server may determine the speed with which each leaf moves. In some examples, a minimum speed may be associated with a minimum moving unit (e.g., "1 cm/s"). In examples, a maximum speed may be associated with a maximum moving unit (e.g., "3 cm/s"). Based clinical contexts, leaves used during IMRT may only move in one direction, whereas leaves used during VMAT may move in two directions. In one illustrative example, with respect to a left and right leaf, 4x4 actions may be performed for IMRT: {0, s}, where s E {1, 2, 3} (0 is stilling and s is the moving speed and 7 x 7 actions for VMAT: {-s, 0, s}, where $s \in \{1, 2, 3\}$ (-s is moving backward). It will be understood that "s" may represent any position integer such that the position integer does not exceed the constraints of the LINAC.

In some embodiments, the experience queue may represent an amount of energy delivered to the patient at a time step and with respect to a given leaf pair (sometimes referred to as a dose rate). For example, at each time step during delivery of energy to the patient, the analytics server may determine a dose rate d. In this example, the analytics server may determine the dose rate based on the analytics server providing data associated with the position of the leaves of the LINAC at one or more time steps, a power level associated with energy transmission by the LINAC at the one or more time steps, and the position and orientation of the PTV relative to the device of the LINAC emitting energy. In some embodiments, the analytics server may store each sample experience episode in the experience queue in association with the corresponding reference fluence map, cumulative fluence map, and/or leaf positions before, during, and/or after movement of the leaves during the transition.

In some embodiments, one or more sample experience episodes may be associated with a reward. For example, the analytics server may determine one or more rewards for each sample experience episode based on the transitions involved in the sample experience episode. Rewards can include, for example, rewards for sample experience episode where the leaves in the leaf pair to not collide during the transition. In one illustrative example, where a position of a first (e.g., left) leaf is greater than a position of a second (e.g., right) leaf, indicating that the first leaf has collided with the second leaf, the analytics server may assign a lower reward to the sample experience episode. Alternatively, where a position of a first (e.g., left) leaf is less than a position of a second (e.g., right) leaf, indicating that the first leaf has not collided with the second leaf, the analytics server may assign a higher reward to the sample experience episode as compared to the reward assigned for the collision.

In examples, the analytics server may determine rewards based on a comparison of a cumulative fluence represented by a cumulative fluence map with a target fluence represented by a target fluence map for a given sample experience episode. For example, higher rewards may be assigned to sample experience episodes where the difference between the cumulative fluence and the target fluence is less than a similar difference of a different sample experience episode. Alternatively, lower rewards may be assigned to sample experience episodes where the difference between the cumulative fluence and the target fluence is greater than a similar difference of a different sample experience episode. For example, where a sample experience episode results in an increased delivery of energy to one or more OARs in proximity to the PTV as compared to other sample experience episodes, the sample experience episode where more energy was delivered to the OAR may be assigned a lower reward. In this way, higher rewards can be assigned to sample experience episodes that result in more precise delivery of energy to at least a portion of a PTV.

In other examples, the analytics server may determine rewards to sample experience episodes based on a speed of motion of each of the leaves of a leaf pair. For example, the analytics server may assign higher rewards to sample experience episodes where one or both leaves move slower during the transition as compared to other sample experience episodes. In this way, the analytics server may increase the significance of sample experience episodes where leaves move slower. This, in turn, can reduce the chances for systematic errors (e.g., mechanical errors, errors in delivering energy to the correct portion of the PTV, and/or the like).

In some embodiments, one or more sample experience episodes in the sample experience queue may be associated with a given PTV. For example, where a training set represents multiple PTVs and corresponding target fluence maps, one or more sample experience episodes may be associated with the delivery of energy to that particular PTV. In this way, groups of sample experience episodes (e.g., representing the operations performed by one or more agents during the delivery of energy to the PTV) may be grouped together prior to sampling.

In some embodiments, the analytics server may sample one or more parameters of the one or more training models of corresponding agents that are represented in the experience queue to optimize a set of models. For example, analytics server may sample one or more parameters of the one or more training models that are represented in the experience queue to determine sets of parameters for models to be used during inference. In this example, each model of the set of models may correspond to a leaf pair of a LINAC. In some embodiments, the analytics server may sample the one or more parameters of the one or more training models to determine a set of models that are associated with an optimized set of parameters based on one or more transitions. For example, where a first training model is associated with a first transition having a first reward and a second training model is associated with a second transition having a second reward that is less than the first reward, the analytics server may determine a model (e.g., one or more weights of the model) based on one or more weights of the first training model. In this way, the analytics server may select parameters for each model of the set of models that optimize the output of each model. In some embodiments, once the model is optimized (e.g., converges such that performance of the model stabilizes), the analytics server an output the model. In some embodiments, the analytics server may sample the one or more parameters of the one or more training models that are represented in the experience queue to optimize a set of models, where the set of models includes a model associated with each scale.

In the preliminary experiments, based on data for patients having prostate cancer and head-and-neck cancer, a total number of plans for each site were developed (as referenced in Table 1 (below), data are filtered due to missing data, quality issue, and incompatibility with environment/software).

Table 1. The number of plans (e.g., patients) of different sites

|  | prostate cancer | Head-and-neck |
| --- | --- | --- |
| IMRT plan | 416 | 0 |
| VMAT plan | 555 | 1157 |

Each plan has several fluence maps. For IMRT plans, each angle corresponded to one fluence map, and each plan usually had 4-10 angles. For VMAT plans, the number of fluences depended on the scale. For example, given one arc in VMAT with 180 control points, if the scale was 18 (18 control points forms a fluence map), the arc could at least generate 180 / 18 = 10 samples for testing. During the training, the data was augmented by randomly selecting the start control points. In experiments, 10% of the plans were used as testing.

Leaf sequencing can be evaluated by: 1) comparing the target fluence and predicted fluence map (generated by predicted leaf sequence), 2) comparing the DVHs of downstream dose calculation with Acuros XB. The error of 1) is calculated by: $Error = \sum_{i=1}^{L} \left\| F_i - \hat{F}_i \right\|_2 / \left\| F_i \right\|_2$, $F \in R^{X \times Y}$, where $F$ and $\hat{F}$ is the target and predicted fluences, and Yis the fluence sizeof single leaf pair and X is the number of leaf pairs of the fluence. The preliminary experiments on both prostate and head-and-neck sites are as follows:

Table 2. Quantitative results in prostate and head-and-neck sites of VMAT

|  | prostate cancer | head and neck |
| --- | --- | --- |
| Error | 0.042 | 0.149 |

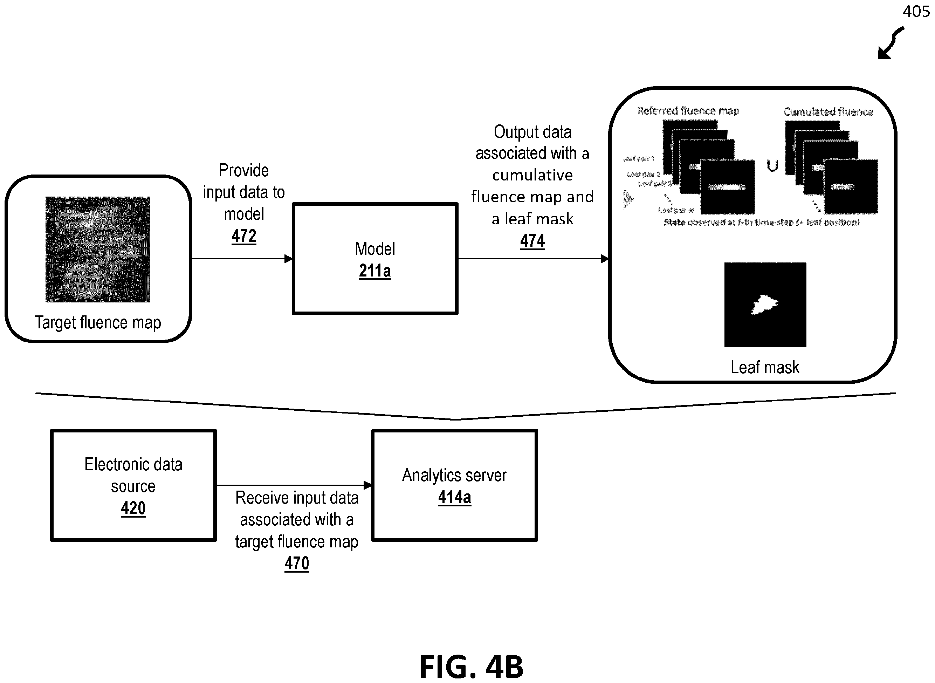

Referring now to **FIGS. 4B-4E,** illustrated is a flowchart of an implementation **405** relating to a process for generating fluence maps when planning radiation therapy. As illustrated in **FIGS. 4B-4E,** implementation **405** includes an electronic data source **420** (which may be the same as, or similar to, electronic data source **120** of **FIG. 1**), an end-user device **440** (which may be the same as, or similar to, the end user device **140** of **FIG. 1**), and an analytics server **414a** (which may be the same as, or similar to, the analytics server **114a** of **FIG. 1).**

At operation **470,** the analytics server **414a** receives data associated with a target fluence map from the electronic data source **420.** In some implementations, the target fluence map may be generated using one or more of the techniques described herein with respect to **FIGS. 3A-3D.** At operation **472,** the analytics server **414a** provides the input data associated with the target fluence map to a model **211a.** In some implementations, the model **211a** may include one or more sub-models, each sub-model corresponding to a component of a medical device (e.g., one or more leaves of a collimator for a medical device that is the same as, or similar to, the medical device **160** of **FIG. 1).** In some implementations, the model and the one or more sub-models may be trained as described herein.

At operation **474,** the analytics server **414a** causes the model **211a** to output data associated with a cumulative fluence map and a leaf mask. In some implementations, the cumulative fluence map is associated with a cumulative amount of energy delivered to the PTV of the patient, and the leaf mask is associated with the position of each leaf of the medical device at a point in time.

At operation **476,** the analytics server **414a** provides updated input data to the model **211a.** In some implementations, the analytics server **414a** provides the updated input data to the model based on the analytics server **414a** determining the updated input data. In these implementations, the analytics server **414a** may determine the updated input data based on the data associated with the cumulative fluence map and the leaf mask output by the model **211a** (e.g., at operation **474**). At operation **478** the analytics server **414a** causes the model **211a** to output data associated with an updated cumulative fluence map and an updated leaf mask.

At operation **480,** the analytics server **414a** determines that the difference between the updated cumulative fluence map and the target fluence map satisfies a threshold amount. In some implementations, the analytics server **414a** determines that the difference between the updated cumulative fluence map and the target fluence map satisfies the threshold amount based on the analytics server **414a** comparing the intensity values of energy per unit area of the updated cumulative fluence map and an intensity value of energy per unit area of the target fluence map.

At operation **482,** the analytics server **414a** determines a leaf sequence. In some implementations, the analytics server **414a** determines the leaf sequence based on the leaf mask and the updated leaf mask. For example, the analytics server **414a** may determine the leaf sequence based on the positions of each of the leaves as represented by the leaf mask and the positions of each of the leaves as represented by the updated leaf mask. In some implementations, the analytics server **414a** also determines one or more power levels at which to deliver energy that correspond to each leaf mask. At operation **484,** the analytics server **414a** outputs data associated with the leaf sequence. In some implementations, the data associated with the leaf sequence may be configured to cause control of the medical device when delivering energy to the patient.

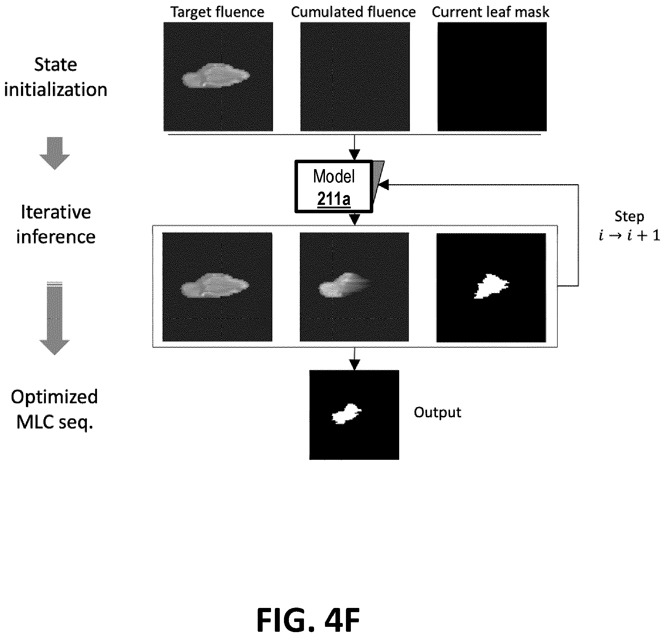

**FIG. 4F** illustrates one example iteration of the implementation of **FIGS. 4B-4E.**

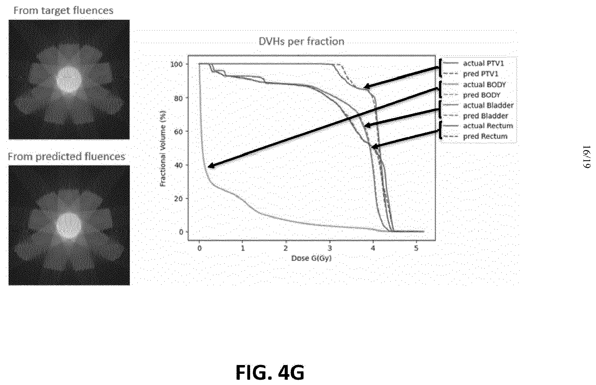

**FIG. 4G** illustrates an example comparison of target (e.g., cumulative) fluences delivered to a patient and predicted fluences delivered to a patient, in accordance with an embodiment.

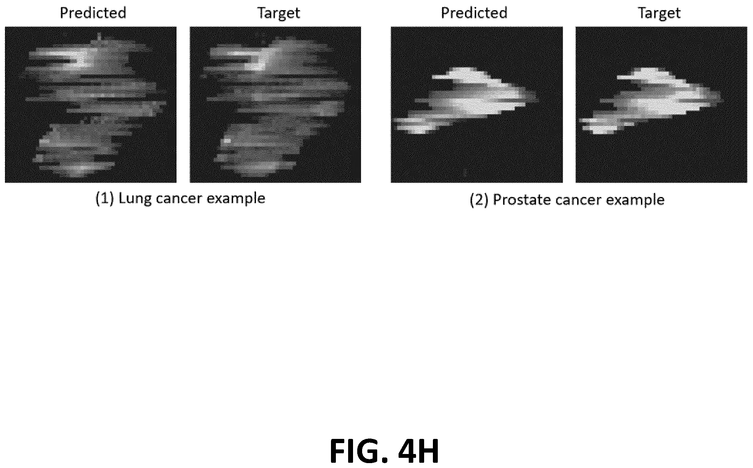

**FIG. 4H** illustrates example pairs of predicted fluence maps and target (e.g., cumulative) fluence maps, in accordance with an embodiment.

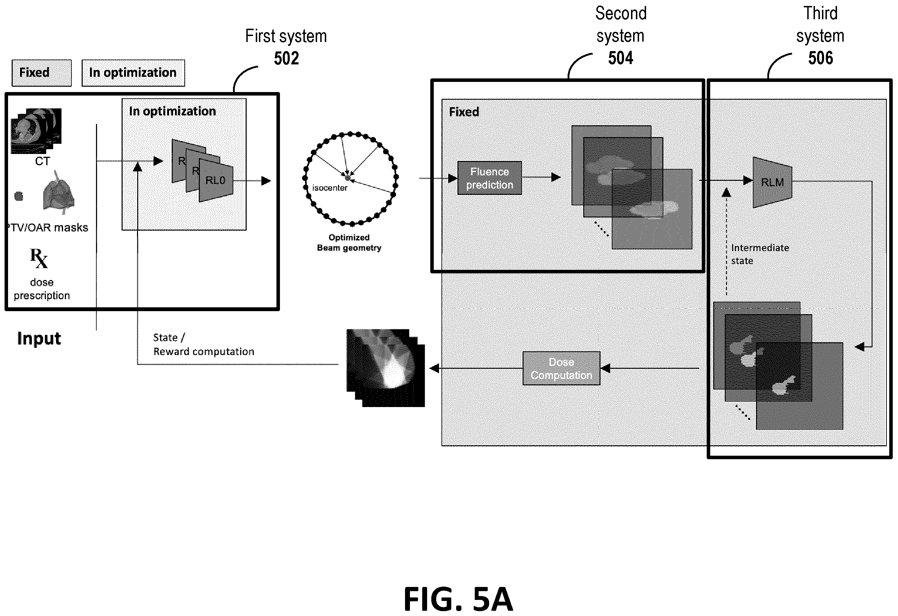

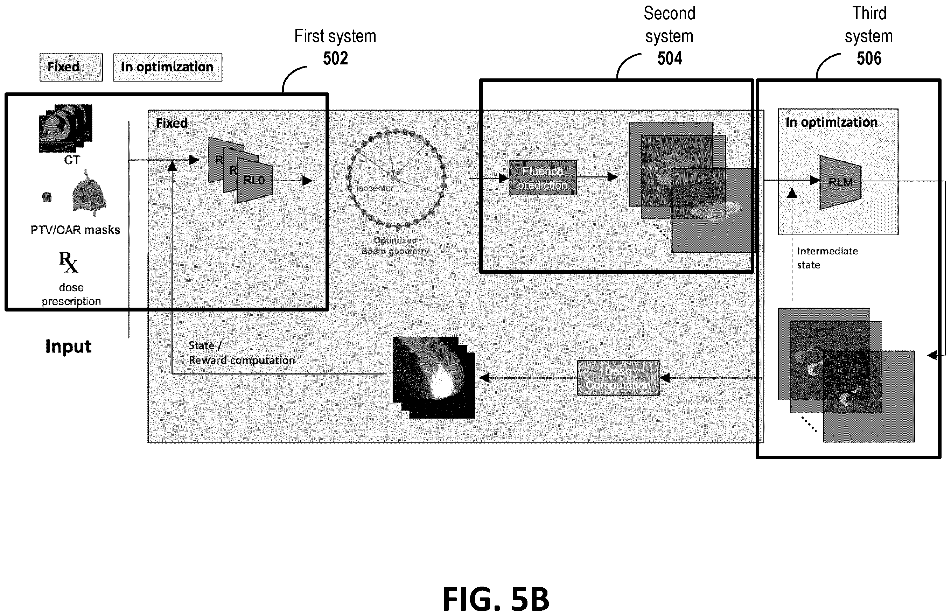

**FIGS. 5A and 5B** illustrate examples training pipelines involving multiple systems, in accordance with an embodiment. As will be described, training of the one or more models described herein may be performed in an end-to-end fashion. More specifically, as discussed with respect to **FIGS. 2A-2D,** one or more models described therein may be used when converting planning target volumes into one or more sets of beam positions (which can be described with respect to the beam angles formed by the beams during energy delivery to the patient) and beam strengths. With respect to **FIGS. 3A-3D,** a different model may be used to convert beams (e.g., beams as output by the one or more models described with respect to **FIGS. 2A-2D)** into a fluence map. And with respect to **FIGS. 4A-4E,** another different model may be used to convert a target fluence map (which may be the same as, or similar to, the fluence map output by the model of **FIGS. 3A-3D)** into a leaf sequence. These models may be trained in such an end-to-end fashion so as to improve the overall performance of a system that is configured to initially receive a planning target volume and provide as an output a leaf sequence to control a LINAC during treatment of a patient.

With continued reference to **FIGS. 5A and 5B,** illustrated is the assembly of multiple models (referred to as an ensemble) when processing data associated with planning target volumes and radiation maps to generate treatment plans that can be executed using a medical device as described herein (e.g., a medical device that is the same as, or similar to, the medical device **160** of **FIG. 1).** As illustrated, a first system **502** may include one or more devices capable of receiving data associated with planning target volumes (e.g., data associated with CT scans, PTV and OAR masks) and determining one or more sets of beam angles and corresponding beam strengths. A second system **504** may include one or more devices capable of receiving data associated with a plurality of beams (e.g., data associated with the one or more beam angles and corresponding beam strengths as discussed with respect to the first system **502)** and determining a fluence map. A third system **506** may include one or more devices capable of receiving data associated with a fluence map (e.g., a target fluence map) and determining a leaf sequence.

In some embodiments, the first system **502** may include one or more systems or devices that are the same as, or similar to, one or more systems or devices described with respect to **FIGS. 2A-2D;** the second system **504** may include one or more systems or devices that are the same as, or similar to, one or more systems or devices described with respect to **FIGS. 3A-3D;** and the third system **506** may include one or more systems or devices that are the same as, or similar to, one or more systems or devices described with respect to **FIGS. 4A-4H.**

With continued reference to FIGS. **5A and 5B,** during training, the first system **502** (e.g., one or more models implemented by the first system **502)** may be trained (as described with respect to FIGS. **2A-2D,** above) until the first system **502** converges. In some embodiments, when the first system **502** is pretrained or after the first system **502** converges during training, the second system **504** (e.g., one or more models implemented by the second system **504)** may be trained (as described with respect to **FIGS. 3A-3D,** above) until the second system **504** converges. In some embodiments, the first system **502** and the second system **504** may be trained concurrently based on the inputs and outputs of each respective system. For example, the first system **502** or the second system **504** may be fixed (e.g., not trained) while the other of the first system **502** or the second system **504** is trained. Once the first system **502** or the second system **504** are trained, respectively, they may be locked (sometimes referred to as being "fixed") and the other of the first system **502** or the second system **504** trained. In some embodiments, the second system **504** and/or the third system **506** may be fixed while the first system **502** is trained. In some embodiments, the first system **502** or the second system **504** may be fixed (*see* FIG. 5B) while the third system **506** is trained. It will be understood that when one system is fixed, the others may receive, process, and provide outputs that eventually result in 3D dose computations. These 3D dose computations may then be compared to originally-received 3D dose computations and the difference used to update one or more weights of one or more not fixed systems to improve the overall performance of the respective systems. In this way, through fixing various systems during the end-to-end training process, the systems may coordinate with one another to holistically search for optimal solutions during the training process.

In some embodiments, the first system **502,** the second system **504** or the third system **506** are pretrained. For example, the first system **502,** the second system **504** or the third system **506** may be pretrained by training each system individually based on the techniques described with respect to **FIGS. 2A-2D, FIGS. 3A-3D,** and **FIGS. 4A-4H,** respectively. In an example, the first system **502** and/or the second system **504** may be pretrained and the pretrained model used to train the other during end-to-end training of the models involved in the first system **502,** the second system **504,** and/or the third system **506.** In some embodiments, when training the second system **504** and/or the third system **506,** the first system **502** may implement a physics-based dose calculation system.

In some embodiments, a fluence prediction module and a dose prediction module are used. The fluence prediction module may receive, as input, CT scans, PTV masks, OAR masks (as provided to the first system **502),** and beam geometry (as output by the first system **502**), and the fluence prediction module may output fluence maps representing each corresponding beam angle (for IMRT) or each subfield (for VMAT). In this way, a fluence prediction module may be used in place of, or in coordination with, the first system 502 when training the second system **504** and/or the third system **506.**

In some embodiments, the dose computation module receives the fluence maps (which may be represented as combinations of leaf sequences and/or corresponding power levels output by the third system **506)** and outputs 3D dose distributions (e.g., as represented by radiation maps). The quality of 3D dose distributions is evaluated (e.g., compared to a predetermined dose prescription) and the difference between the 3D dose distributions is used to train the system (e.g., to update one or more weights of models implemented by the first system **502,** the second system **504,** and/or the third system **506).**

Some non-limiting embodiments of the present disclosure are described herein in connection with a threshold. As described herein, satisfying a threshold may refer to a value being greater than the threshold, more than the threshold, higher than the threshold, greater than or equal to the threshold, less than the threshold, fewer than the threshold, lower than the threshold, less than or equal to the threshold, equal to the threshold, and/or the like.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein may be applied to other embodiments without departing from the spirit or scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope and spirit being indicated by the following claims.

**Claims**

1. A system comprising:
   at least one processor programmed to:

   receive data associated with a first planning target volume and a first radiation map, the first planning target volume representing an area of a body of a patient and a target mask, the target mask representing a portion of the area of the body of the patient to receive radiation;
   provide the first planning target volume and the first radiation map to a first model to cause the first model to output data associated with at least one first beam position and least one first beam strength, the at least one first beam position corresponding to the at least one first beam strength,
   generate a second radiation map based on the at least one first beam position, the at least one first beam strength, and the first radiation map;
   provide the first planning target volume and the second radiation map to a second model to cause the second model to output data associated with at least one second beam position and least one second beam strength; and
   transmit data associated with the at least one second beam position and the least one second beam strength to cause a medical device to deliver radiation to the patient.

2. The system of claim 1, wherein the first model is trained to output data associated with first beam positions at a first scale,

   wherein the second model is trained to output data associated with second beam positions at a second scale, and
   wherein the first radiation map is associated with a first resolution that is less than a resolution of the second radiation map.

3. The system of claim 2, wherein the first scale is associated with a first set of candidate angles within a candidate angle space,

   wherein the second scale is associated with a second set of candidate angles within the candidate angle space, and
   wherein the first set of candidate angles are associated with angle measurements that are greater than angle measurements associated with the second set of candidate angles.

4. The system of claim 3, wherein a beam associated with the second set of candidate angles corresponds to a beam associated with the first set of candidate angles.

5. The system of any one of claims 1 to 4, wherein the first model is trained based on a reinforcement learning agent and training data associated with previously-treated patients.

6. The system of claim 5, wherein the training data represents planning target volumes and goal radiation maps that correspond to the previously-treated patients; and

   wherein, when training, the at least one processor is further programmed to:
   for each previous-treated patient of the previously-treated patients:

   generate data associated with beam positions and beam strengths based on a plurality of reinforcement learning agents, each reinforcement learning agent associated with a training model,
   provide the data associated with beam positions and beam strengths to a simulator to generate a set of simulated outputs,
   determine rewards for each reinforcement learning agent of the plurality of reinforcement learning agents based on the corresponding simulated outputs and the goal

   radiation map, and
   select the first model based on the rewards for each reinforcement learning agent; and,
   optionally, wherein the goal radiation maps represent radiation maps that were previously generated based on input by a clinician, the goal radiation maps representing preferences of the clinician when delivering radiation to the previously-treated patients.

7. The system of any one of claims 1 to 6, wherein the medical device comprises a linear accelerator, LINAC.

8. A method comprising:

receiving, by at least one processor, data associated with a first planning target volume and a first radiation map, the first planning target volume representing an area of a body of a patient and a target mask, the target mask representing a portion of the area of the body of the patient to receive radiation;
providing, by the at least one processor, the first planning target volume and the first radiation map to a first model to cause the first model to output data associated with at least one first beam position and least one first beam strength, the at least one first beam position corresponding to the at least one first beam strength,
generating, by the at least one processor, a second radiation map based on the at least one first beam position, the at least one first beam strength, and the first radiation map;
providing, by the at least one processor, the first planning target volume and the second radiation map to a second model to cause the second model to output data associated with at least one second beam position and least one second beam strength.

9. The method of claim 8, wherein the first model is trained to output data associated with first beam positions at a first scale, and
wherein the second model is trained to output data associated with second beam positions at a second scale.

10. The method of claim 9, wherein the first scale is associated with a first set of candidate angles within a candidate angle space,

wherein the second scale is associated with a second set of candidate angles within the candidate angle space, and
wherein the first set of candidate angles are associated with angle measurements that are greater than angle measurements associated with the second set of candidate angles.

11. The method of claim 10, wherein a beam associated with the second set of candidate angles corresponds to a beam associated with the first set of candidate angles.

12. The method of any one of claims 8 to 11, wherein the first model is trained based on a reinforcement learning agent and training data associated with previously-treated patients.

13. The method of claim 12, wherein the training data represents planning target volumes and goal radiation maps that correspond to the previously-treated patients;
the method further comprising:
for each previous-treated patient of the previously-treated patients:

generating data associated with beam positions and beam strengths based on a plurality of reinforcement learning agents, each reinforcement learning agent associated with a training model,
providing the data associated with beam positions and beam strengths to a simulator to generate a set of simulated outputs,
determining rewards for each reinforcement learning agent of the plurality of reinforcement learning agents based on the corresponding simulated outputs and the goal radiation map, and
selecting the first model based on the rewards for each reinforcement learning agent.

14. The method of claim 13, wherein the goal radiation maps represent radiation maps that were previously generated by the at least one processor based on input by a clinician, the goal radiation maps representing preferences of the clinician when delivering radiation to the previously-treated patients.

15. A non-transitory machine-readable medium having instructions stored thereon that, when executed by at least one processor, cause the at least one processor to perform operations comprising:

receiving data associated with a first planning target volume and a first radiation map, the first planning target volume representing an area of a body of a patient and a target mask, the target mask representing a portion of the area of the body of the patient to receive radiation;

providing the first planning target volume and the first radiation map to a first model to cause the first model to output data associated with at least one first beam position and least one first beam strength, the at least one first beam position corresponding to the at least one first beam strength,

generating a second radiation map based on the at least one first beam position, the at least one first beam strength, and the first radiation map;

providing the first planning target volume and the second radiation map to a second model to cause the second model to output data associated with at least one second beam position and least one second beam strength;

transmitting data associated with the at least one second beam position and the least one second beam strength to cause a medical device to deliver radiation to the patient; and,

optionally, wherein the instructions stored on the machine-readable medium, when executed by the at least one processor, cause the at least one processor to perform the method of any one of claims 8 to 14.

**FIG. 1**

EP 4 596 039 A1

200

Receive data associated with a first planning target volume and a first radiation map. **202**

Provide the first planning target volume and the first radiation map to a first model to cause the first model to output data associated with at least one first beam position and at least one first beam strength. **204**

Generate a second radiation map based on the at least one first beam position, the at least one first beam strength, and the first radiation map. **206**

Provide the first planning target volume and the second radiation map to a second model to cause the second model to output data associated with at least one second beam position and least one second beam strength. **208**

Transmit data associated with the at least one second beam position and the at least one second beam strength to cause a linear accelerator (LINAC) to deliver radiation to a patient. **210**

## FIG. 2A

**FIG. 2B**

EP 4 596 039 A1

FIG. 2C

EP 4 596 039 A1

Output data
associated with at
least one second
beam position and
at least one second
beam strength
**282**

Provide data to
model
**280**

| Model **211b** |

Data associated
with a first planning
target volume

Data associated
with a second
radiation map

Generate a second
radiation map
**278**

| Analytics server **214a** |

Transmit data
**284**

205

**FIG. 2D**

EP 4 596 039 A1

Receive data associated with a plurality of beams. **302**

Generate beam eye view (BEV) images based on the plurality of beams. **304**

Determine a digitally reconstructed radiograph (DRR) based on concatenating the BEV images. **306**

Provide the DRR to a model to cause the model to generate an output, the output associated with a fluence map. **308**

**FIG. 3A**

BEV image 1

• • •

BEV image n

Electronic data source 320 → Receive data associated with a plurality of beams 370 → Analytics server 314a → Generate beam eye view (BEV) images 372

## FIG. 3B

EP 4 596 039 A1

305

BEV image 1      +      BEV image n

Analytics server
314a

Determine a
digitally
reconstructed
radiograph (DRR)
374

**FIG. 3C**

FIG. 3D

Receive input data associated with a target fluence map. **402**

Provide the input data to a model to cause the model to generate an output, the output representing a cumulative fluence map and a leaf mask. **404**

Generate updated input data associated with the target fluence map, the cumulative fluence map, and the leaf mask. **406**

Provide the updated input data to the model to cause the model to generate an updated output, the updated output representing an updated cumulative fluence map and an updated leaf mask. **408**

Determine that the difference between the updated cumulative fluence map and the target fluence map satisfies a second threshold amount. **410**

Determine a leaf sequence based on the leaf mask and the updated leaf mask. **412**

400

# FIG. 4A

EP 4 596 039 A1

EP 4 596 039 A1

405

Provide
input data to
model
**472**

Output data
associated with a
cumulative
fluence map and
a leaf mask
**474**

Referred fluence map        Cumulated fluence

Leaf pair 1
Leaf pair 2
Leaf pair 3

Leaf pair N

**State** observed at *i*-th time-step (+ leaf position)

Leaf mask

Model
**211a**

Target fluence map

Electronic data
source
**420**

Receive input data
associated with a
target fluence map
**470**

Analytics server
**414a**

**FIG. 4B**

**FIG. 4C**

405

Target fluence map

Cumulative fluence map

Determine that the difference
between the updated
cumulative fluence map and
the target fluence map satisfies
a threshold amount
**480**

Analytics server
**414a**

## FIG. 4D

EP 4 596 039 A1

EP 4 596 039 A1

405

Determine a leaf
sequence
482

Analytics server
414a

Output data
associated with
the leaf
sequence
484

**FIG. 4E**

FIG. 4F

EP 4 596 039 A1

From target fluences

From predicted fluences

DVHs per fraction

FIG. 4G

(2) Prostate cancer example

(1) Lung cancer example

FIG. 4H

FIG. 5A

**FIG. 5B**

| | Europäisches Patentamt European Patent Office Office européen des brevets | **EUROPEAN SEARCH REPORT** | **Application Number** EP 25 15 4462 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/191158 A1 (BOSE SUPRATIK [US] ET AL) 22 June 2023 (2023-06-22)<br>* paragraphs [0061], [0062], [0084] - [0089], [0108] - [0123], [0145] - [0164], [0179], [0180] *<br>* figures 1-3,10 * | 1-4, 8-11,15 | INV.<br>A61N5/10 |
| X | US 2022/414525 A1 (PRINC SANTIAGO GASPAR [FI] ET AL) 29 December 2022 (2022-12-29)<br>* paragraphs [0006] - [0026], [0053], [0073] *<br>* figures 1,2,3 * | 1,5-8, 12-15 | |
| A | US 2022/088410 A1 (HIBBARD LYNDON STANLEY [US]) 24 March 2022 (2022-03-24)<br>* abstract *<br>* figures 1,9,15 * | 1-15 | |

TECHNICAL FIELDS SEARCHED (IPC)

A61N

The present search report has been drawn up for all claims

| Place of search | Date of completion of the search | Examiner |
|---|---|---|
| Munich | 21 May 2025 | Kretschmann, Jana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 4462

This annex lists the patent family members relating to the patent documents cited in the above-mentioned European search report.
The members are as contained in the European Patent Office EDP file on
The European Patent Office is in no way liable for these particulars which are merely given for the purpose of information.

21-05-2025

| Patent document cited in search report | Publication date | Patent family member(s) | | Publication date |
|---|---|---|---|---|
| US 2023191158 A1 | 22-06-2023 | CN | 115968497 A | 14-04-2023 |
| | | US | 2023191158 A1 | 22-06-2023 |
| | | WO | 2022036631 A1 | 24-02-2022 |
| US 2022414525 A1 | 29-12-2022 | CN | 117561102 A | 13-02-2024 |
| | | EP | 4359070 A1 | 01-05-2024 |
| | | US | 2022414525 A1 | 29-12-2022 |
| | | WO | 2022268576 A1 | 29-12-2022 |
| US 2022088410 A1 | 24-03-2022 | CN | 116391234 A | 04-07-2023 |
| | | EP | 4200872 A1 | 28-06-2023 |
| | | US | 2022088410 A1 | 24-03-2022 |
| | | WO | 2022061324 A1 | 24-03-2022 |

EPO FORM P0459

For more details about this annex : see Official Journal of the European Patent Office, No. 12/82

**REFERENCES CITED IN THE DESCRIPTION**

**Non-patent literature cited in the description**

- **SCHULMAN et al.** *Proximal Policy Optimization Algorithms*, https://arxiv.org/abs/1707.06347